(19) Europäisches Patentamt
European Patent Office
Office européen des brevets

(11) EP 0 655 908 B1

(12) **FASCICULE DE BREVET EUROPEEN**

(45) Date de publication et mention de la délivrance du brevet:
**07.01.1999 Bulletin 1999/01**

(21) Numéro de dépôt: **94919743.8**

(22) Date de dépôt: **21.06.1994**

(51) Int. Cl.[6]: **A61K 7/42**, A61K 7/48

(86) Numéro de dépôt international:
**PCT/FR94/00751**

(87) Numéro de publication internationale:
**WO 95/00111 (05.01.1995 Gazette 1995/02)**

(54) **COMPOSITIONS COSMETIQUES FILTRANTES CONTENANT UN AGENT HYDROPHILE COMPORTANT AU MOINS UN RADICAL ACIDE SULFONIQUE**

FILTRIERENDE KOSMETISCHE ZUSAMMENSETZUNG ENTHALTEND EIN HYDROPHILIERUNGSMITTEL MIT MINDESTENS EINEM SULFONSÄUREREST

COSMETIC FILTERING COMPOSITIONS CONTAINING A HYDROPHILIC AGENT WITH AT LEAST ONE SULPHONIC ACID RADICAL

(84) Etats contractants désignés:
**AT BE CH DE DK ES FR GB GR IE IT LI LU MC NL PT SE**

(30) Priorité: **21.06.1993 FR 9307447**

(43) Date de publication de la demande:
**07.06.1995 Bulletin 1995/23**

(73) Titulaire: **L'OREAL**
**75008 Paris (FR)**

(72) Inventeur: **HANSENNE, Isabelle**
**F-75017 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 275 719**
**EP-A- 0 390 682**
**EP-A- 0 445 700**
**EP-A- 0 518 772**
**CH-A- 472 220**
**DE-A- 2 407 733**
**GB-A- 2 025 957**
**GB-A- 2 121 801**
**GB-A- 2 185 019**
**US-A- 3 670 074**

- **DATABASE WPI Week 9311, Derwent Publications Ltd., London, GB; AN 93-088577 & JP,A,5 032 532 (SHISEIDO CO LTD) 9 Février 1993 cité dans la demande**
- **PATENT ABSTRACTS OF JAPAN vol. 17, no. 185 (C-1047) 12 Avril 1993 & JP,A,04 338 317 (KAO CORP) 25 Novembre 1992**

Remarques:
Le dossier contient des informations techniques présentées postérieurement au dépôt de la demande et ne figurant pas dans le présent fascicule.

EP 0 655 908 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne des compositions cosmétiques substantives et résistantes à l'eau, destinées à protéger la peau et les cheveux du rayonnement ultraviolet. Elle concerne plus particulièrement des compositions cosmétiques substantives et résistantes à l'eau contenant un filtre UV hydrophile comportant au moins un radical acide sulfonique. L'invention concerne encore leur utilisation pour la protection de la peau et des cheveux contre le rayonnement ultraviolet.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage ; ce rayonnement UV-B doit donc être filtré.

On sait également que les rayons UV-A, de longueurs d'onde comprises entre 320 et 400 nm, en provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Au sens de la présente invention, la substantivité peut être caractérisée par le caractère hydrophobe de l'association filtrante c'est-à-dire sa non dissociation dans l'eau ; la résistance à l'eau peut être définie par la stabilité au cours du temps après douche ou baignade de l'indice de protection dans l'UVA et/ou l'UVB.

On a déjà proposé d'améliorer la substantivité et la résistance à l'eau des compositions filtrantes, en les formulant soit avec des polymères (voir notamment brevet US 5.041.281 et demande de brevet JP-A- 05 032532), soit dans un support émulsion eau dans huile en présence d'émulsionnants d'un HLB (hydrophilie-lipophilie-balance) variant de 1 à 7 (voir notamment brevet US 5.047.232).

Ces deux techniques permettent effectivement d'améliorer la résistance à l'eau des compositions lorsque celles-ci renferment des filtres lipophiles. Il n'en est cependant pas de même avec les compositions renfermant des filtres hydrophiles, acides notamment, car ceux-ci disparaissent à l'eau, par baignade en mer ou en piscine, sous la douche ou lors de la pratique de sports nautiques ; ainsi les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

Dans la demande de brevet EP A-0275719, on a cherché à rendre substantives et résistantes à l'eau des compositions solaires renfermant des filtres acides, en associant ceux-ci à une amine grasse.

Ce type de solution est dans certains cas insatisfaisant du fait de l'impossibilité de combiner certains filtres acides avec des amines grasses et du fait aussi que les amines grasses peuvent provoquer des allergies de contact comme cela est décrit dans l'ouvrage "Adverse reactions to cosmetics" (Anton de Cornelis de Groot - Ed. Rijksuniversiteit Groningen, 1988) chapitre 5, p. 170 et suivantes.

Le document D10 divulgue des compositions filtrantes contenant une silicone aminée associée à des filtres UV notamment du type acide p-amino benzoique.

Or, la demanderesse a maintenant découvert une nouvelle composition cosmétique filtrante, du type mettant en oeuvre un filtre hydrophile comportant au moins un groupement acide sulfonique, qui pallie les inconvénients de l'art antérieur et qui présente de plus une substantivité et une résistance à l'eau améliorées.

L'indice de protection ou IP peut s'exprimer par le rapport du temps d'irradiation nécessaire pour atteindre le seuil érythématogène avec le filtre UV au temps nécessaire pour atteindre le seuil érythématogène sans filtre UV.

L'amélioration de ces propriétés est particulièrement intéressante dans le cas des compositions solaires sous forme d'émulsions huile dans eau.

Dans le cas des émulsions huile dans eau ou eau dans huile, les filtres hydrophiles sont présents dans la phase aqueuse, les filtres lipophiles, dans la phase grasse. Or, les émulsions huile dans eau sont bien plus appréciées par le consommateur que les émulsions eau dans huile, pour leur toucher agréable (voisin de l'eau) et leur présentation sous forme de lait ou de crème non gras ; cependant, elles perdent également plus facilement leur efficacité en protection UV dès lors qu'elles viennent en contact avec l'eau ; cette perte en indice de protection par élimination à l'eau du filtre hydrophile étant d'autant plus marquée que l'association filtrante lipophile-hydrophile, présente dans la composition, est synergique au plan de l'indice de protection.

La présente invention a ainsi pour objet une composition cosmétique filtrante, caractérisée en ce qu'elle comprend, dans un support cosmétiquement acceptable, au moins un agent hydrophile filtrant le rayonnement ultraviolet comportant au moins un radical acide sulfonique ($-SO_3H$) et au moins un dérivé siliconé aminofonctionnel.

On notera ici que la présente invention ne convient pas aux compositions qui contiendraient des filtres hydrophiles à radical carboxylique, la rémanence à l'eau de ce type de filtres n'étant en effet pas améliorée en procédant à l'association conforme à l'invention.

Comme exemple de filtres acides contenant au moins un groupe $SO_3H$, on peut citer les dérivés sulfoniques du 3-benzylidène 2-bornanone et notamment ceux de formules (I), (II), (III), (IV), et (V) suivantes :

*Formule (I) :*

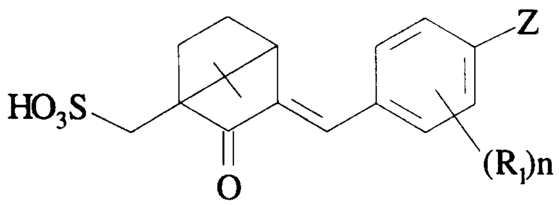

(I)

dans laquelle :

- Z désigne un groupement

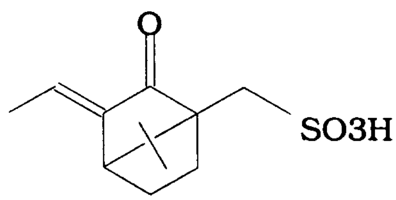

- n est égal à 0 ou est un nombre entier compris entre 1 et 4(0 ≤n≤ 4)
- $R_1$, représente un ou plusieurs radicaux alkyle ou alkoxy, identiques ou différents, linéaires ou ramifiés, contenant de 1 à 4 atomes de carbone environ.

Un composé de formule I particulièrement préféré est celui correspondant à n = 0 : l'acide benzène 1,4 [di(3-méthylidènecampho 10-sulfonique)].

*Formule (II) :*

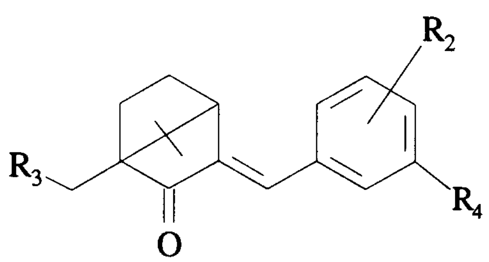

(II)

dans laquelle :

- $R_2$ désigne un atome d'hydrogène, un atome d'halogène, un radical alkyle contenant de 1 à 4 atomes de carbone environ ou un radical $-SO_3H$.

- $R_3$ et $R_4$ désignent un atome d'hydrogène ou un radical $-SO_3H$ au moins un des radicaux $R_2$, $R_3$ ou $R_4$ désignant le radical $-SO_3H$, $R_2$ et $R_4$ ne pouvant désigner simultanément un radical-$SO_3H$.

On peut citer comme exemples particuliers les composés suivants de formule II dans laquelle :

- $R_2$ désigne le radical $-SO_3H$ en position para du benzylidènecamphre et $R_3$ et $R_4$ désignent chacun un atome d'hydrogène, c'est à dire l'acide 4-(3-méthylidènecamphre) benzène sulfonique.
- $R_2$ et $R_4$ désignent chacun un atome d'hydrogène et $R_3$ désigne un radical $-SO_3H$, c'est-à-dire l'acide 3-benzylidène campho-10-sulfonique.
- $R_2$ désigne un radical méthyle en position para du benzylidènecamphre, $R_4$ un radical $-SO_3H$ et $R_3$ un atome d'hydrogène, c'est-à-dire l'acide 2-méthyl 5-(3-méthylidènecamphre) benzène sulfonique.
- $R_2$ désigne un atome de chlore en position para du benzylidènecamphre, $R_4$ un radical $-SO_3H$ et $R_3$ un atome d'hydrogène, c'est-à-dire l'acide 2-chloro 5-(3-méthylidènecamphre) benzène sulfonique.
- $R_2$ désigne un radical méthyle en position para du benzylidènecamphre, $R_4$ désigne un atome d'hydrogène et $R_3$ désigne un radical $-SO_3H$, c'est-à-dire l'acide 3-(4-méthyl) benzylidène campho 10-sulfonique.

*Formule (III):*

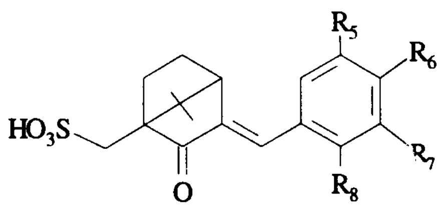

(III)

dans laquelle :

- $R_5$ et $R_7$ désignent un atome d'hydrogène, un radical hydroxyle, un radical alkyle ou alcoxy, linéaire ou ramifié, contenant de 1 à 8 atomes de carbone environ, l'un au moins des radicaux $R_5$ et $R_7$ représentant un radical hydroxyle, alkyle ou alcoxy,
- $R_6$ et $R_8$ désignent un atome d'hydrogène, un radical hydroxyle, l'un au moins des radicaux $R_6$ et $R_8$ désignent le radical hydroxyle, sous réserve que lorsque $R_5$ et $R_8$ désignent un atome d'hydrogène et que $R_6$ désigne un radical hydroxyle, $R_7$ ne désigne pas un radical alcoxy ou un atome d'hydrogène.

On peut citer comme exemples particuliers les composés suivants de formule (III) dans laquelle :

- $R_5$ est un radical méthyle, $R_6$ un atome d'hydrogène, $R_7$ un radical tertiobutyle, $R_8$ un radical hydroxyle, c'est-à-dire l'acide (3-t-butyl 2-hydroxy 5-méthyl) benzylidène campho-10-sulfonique.
- $R_5$ est un radical méthoxy, $R_6$ un atome d'hydrogène, $R_7$ un radical tertiobutyle, $R_8$ un radical hydroxyle, c'est-à-dire l'acide (3-t-butyl 2-hydroxy 5-méthoxy) benzylidène campho- 10-sulfonique.
- $R_5$ et $R_7$ désignent chacun un radical tertiobutyle, $R_6$ un radical hydroxyle, $R_8$ un atome d'hydrogène, c'est-à-dire l'acide (3,5-diterbutyl 4-hydroxy) benzylidène campho-10-sulfonique.

*Formule (IV):*

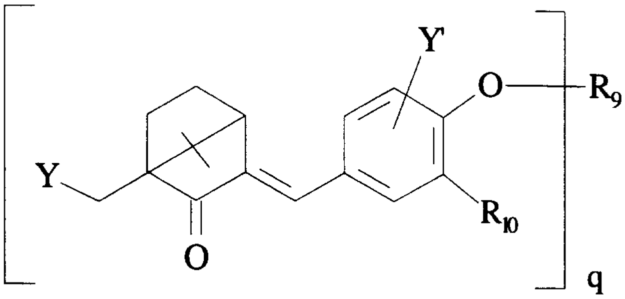

(I V)

dans laquelle :

- $R_9$ désigne un atome d'hydrogène, un radical alkyle, linéaire ou ramifié, contenant de 1 à 18 atomes de carbone environ, un radical alcényle, linéaire ou ramifié, contenant de 3 à 18 atomes de carbone environ, un groupement

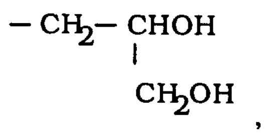

$-(CH_2CH_2O)_n-H$, ou $-CH_2-CHOH-CH_3$,
ou encore un radical divalent : $-(CH_2)_m-$ ou $-CH_2-CHOH-CH_2-$
n étant un nombre entier compris entre 1 et 6($1 \leq n \leq 6$) et m un nombre entier compris entre 1 et 10 ($1 \leq m \leq 10$),

- $R_{10}$ désigne un atome d'hydrogène, un radical alcoxy contenant de 1 à 4 atomes de carbone environ ou un radical divalent - O - relié au radical $R_9$ lorsque celui-ci est divalent lui aussi,

- q désigne un nombre entier égal à 1 ou 2, étant entendu que si q est égal à 2, $R_9$ doit désigner un radical divalent,

- Y et Y' désignent un atome d'hydrogène ou un radical $-SO_3H$, au moins un de ces radicaux Y ou Y' est différent de l'hydrogène.

On peut citer comme exemples particuliers les composés suivants de formule (IV) dans laquelle :

- q est égal à 1, Y et $R_{10}$ désignent chacun un atome d'hydrogène, $R_9$ désigne un radical méthyle, Y' en position 3 désigne un radical $-SO_3H$, c'est-à-dire l'acide 2-méthoxy 5-(3-méthylidènecamphre) benzène sulfonique.

- q est égal à 1, Y désigne un radical $-SO_3H$, Y' un atome d'hydrogène, $R_{10}$ un radical divalent -O- relié à $R_9$ désignant un radical méthylène, c'est-à-dire l'acide 3-(4,5-méthylènedioxy) benzylidène campho-10-sulfonique.

- q est égal à 1, Y désigne un radical $-SO_3H$, Y' et $R_{10}$ désignent tous deux un atome d'hydrogène, $R_9$ désigne un radical méthyle, c'est-à-dire l'acide 3-(4-méthoxy) benzylidène campho-10-sulfonique.

- q est égal à 1, Y désigne un radical $-SO_3H$, Y' un atome d'hydrogène; $R_9$ désigne un radical méthyle, $R_{10}$ désigne un radical méthoxy, c'est-à-dire l'acide 3-(4,5-diméthoxy) benzylidène campho-10-sulfonique.

- q est égal à 1, Y désigne un radical $-SO_3H$, Y' et $R_{10}$ désignent tous deux un atome d'hydrogène; $R_9$ un radical n butyle, c'est-à-dire l'acide 3-(4-n.butoxy) benzylidène campho-10-sulfonique.

- q est égal à 1, Y désigne un radical $-SO_3H$, Y' un atome d'hydrogène; $R_9$ désigne un radical n butyle, $R_{10}$ un radical méthoxy, c'est-à-dire l'acide 3-(4-n.butoxy 5-méthoxy) benzylidène campho-10-sulfonique.

*Formule (V):*

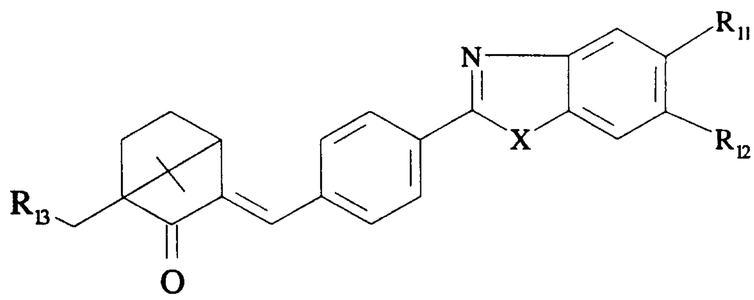

(V)

dans laquelle :

- $R_{11}$ désigne un atome d'hydrogène, un radical alkyle ou alcoxy, linéaire ou ramifié, contenant de 1 à 6 atomes de carbone environ ou un radical $-SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de formule (V) : le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène) phényl] benzimidazole-5-sulfonique.

Les composés de structures (I), (II), (III), (IV), (V) sont respectivement décrits dans le brevet US 4.585.597 et les brevets FR 2.236.515, 2.282.426, 2.645.148, 2.430.938, 2.592.380.

Le filtre à groupement sulfonique peut également être un dérivé sulfonique de benzophénone de formule (VI) :

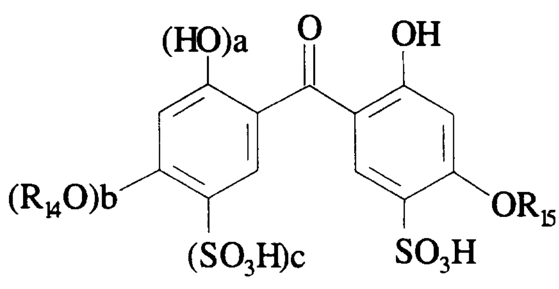

(VI)

dans laquelle :

- $R_{14}$ et $R_{15}$, identiques ou différents, désignent soit un atome d'hydrogène soit un radical alkyle, linéaire ou ramifié, contenant de 1 à 8 atomes de carbone environ.
- a, b et c , identiques ou différents, sont égaux à 0 ou 1.

On peut citer comme exemple particulier de formule (VI) : l'acide 2-hydroxy 4-méthoxybenzophénone 5-sulfonique (composé de formule VI dans laquelle a, b, et c sont égaux à zéro, et $R_{15}$ désigne un radical méthyle).

Le filtre à groupement sulfonique peut encore être un dérivé sulfonique de benzimidazole de formule :

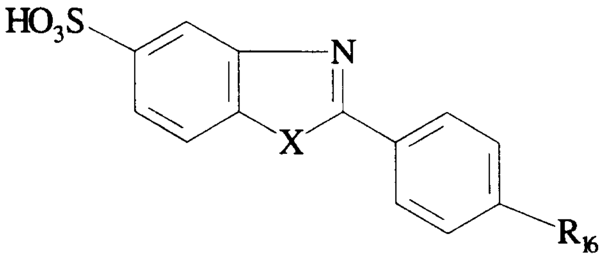

( VII )

dans laquelle :

- X désigne un atome d'oxygène ou un radical -NH-
- $R_{16}$ désigne un atome d'hydrogène, un radical alkyle ou alcoxy, linéaire ou ramifié, contenant de 1 à 8 atomes de carbone environ ou un groupement de formule

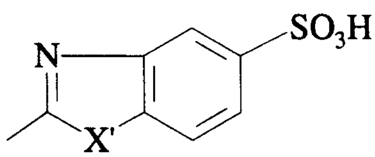

( VIII )

dans laquelle X' représente un atome d'oxygène ou un radical -NH-.

On peut citer comme exemples particuliers les composés suivants de formule (VII) dans laquelle:

- X désigne le radical -NH- et $R_{16}$ désigne un atome d'hydrogène : l'acide 2-phénylbenzimidazole 5-sulfonique.
- X désigne le radical -NH-, $R_{16}$ désigne le groupement de formule (VIII) dans lequel X' désigne le radical -NH- : l'acide benzène 1,4 -di(benzimidazol -2 yl-5-sulfonique).
- X désigne un atome d'oxygène, $R_{16}$ désigne le groupement de formule (VIII) dans lequel X' désigne un atome d'oxygène : l'acide benzène 1,4-di (benzoxazol -2 yl -5-sulfonique).

Les composés de formule VI et VII sont des composés connus pouvant être préparés selon des méthodes classiques décrites dans l'art antérieur.

Le dérivé siliconé aminofonctionnel utilisé dans le cadre de la présente invention est utile en tant que composé hydrophobe neutralisant la fonction acide sulfonique du filtre hydrophile, la neutralisation étant obtenue avec une quantité de dérivé siliconé compatible avec la réalisation d'une composition cosmétique.

Selon un mode de réalisation préférentiel de l'invention le dérivé siliconé aminofonctionnel présente un indice d'amine supérieur à 0,25 meq/g environ. Encore plus préférentiellement, cet indice est supérieur à 0,50 environ.

Dans la composition selon l'invention, le dérivé siliconé aminofonctionnel peut être un dérivé de silicone comportant au moins une fonction amine primaire, secondaire ou tertiaire.

On peut citer parmi eux à titre d'exemples :

(a) les silicones porteurs d'un radical $NH_2$ comme par exemple :

. les polydiméthylsiloxanes (PDMS) $\alpha$, $\omega$, hydroxylées sur la chaîne principale, dénommées dans le dictionnaire CTFA 1991 - 4$^{e}$ éd. AMODIMETHICONE ; on cite par exemple la Silicone Fluid L650 vendue par WACKER

SILICONE, d'indice d'amine 2,7 à 3,2 meq/gramme, la Silicone Fluid L655 de WACKER SILICONE d'indice d'amine 1,3 à 1,45 meq/gramme, la Silicone Fluid L656 de WACKER SILICONE d'indice d'amine 1,2 à 1,4 meq/gramme.

. Les polydiméthylsiloxanes (PDMS) α, ω, alkylées sur la chaîne principale, comme les PDMS α, ω triméthylées telles que celles répondant à l'appellation TRIMETHYLSILYLAMODIMETHICONE dans le dictionnaire CTFA 1991 - 4[e] éd. et parmi elles, on peut citer notamment, 1'$X_2$-8260 vendue par la société DOW CORNING, d'indice d'amine 2,8 meq/gramme ; ou la SLM 55051/3 vendue par la société WACKER, d'indice d'amine 0,47 meq/gramme, les PDMS diméthylalkyle en $C_{12}$, telles que la SLM 23056/1 vendue par la société WACKER, d'indice d'amine 1,2 meq/gramme.

. Les polyméthylalkyl (gras) arylalkylsiloxane α, ω triméthylées, telles que la SLM 23056/2 vendue par la société WACKER, d'indice d'amine 1,3 meq/gramme.

. Des PDMS obtenues par copolymérisation d'un silane difonctionnel et d'un monomère $CH_3$ -Si(OR)$_2$-($CH_2$)$_3$-$NH_2$ où R est un radical éthyle et/ou méthyle, telles que la RHODORSIL Huile PL 1300 vendue par la société RHONE POULENC d'indice d'amine 0,92 meq/gramme.

. Des PDMS dont le radical $NH_2$ est en position α et ω sur un site alkyle telles que les produits TEGOMER A-SI 2120, d'indice d'amine 1,95 meq/gramme et TEGOMER A-SI 2320, d'indice d'amine 0,86 meq/gramme vendus par la société GOLDSCHMIDT.

(b) les silicones porteurs d'un radical -NH- tels que les produits correspondant à la dénomination AMINOBISPROPYLDIMETHICONE dans le dictionnaire CTFA 1991 - 4[e] éd., dont on peut citer l'UCARE SILICONE ALE 56 vendue par la société UNION CARBIDE, d'indice d'amine 0,86 meq/gramme,

(c) les silicones porteurs d'un radical

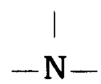

comme le composé de structure :

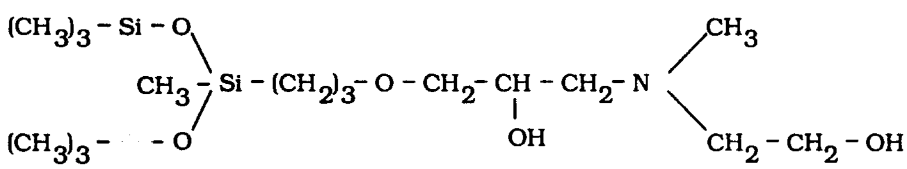

composé connu (RN 56039-74-8) d'indice d'amine égal à 2,58 meq/gramme.

Des exemples de compositions cosmétiques filtrantes préférées comprennent les associations de filtre UV hydrophile à fonction(s) acide(s) sulfonique(s) et de dérivé siliconé aminofonctionnel suivantes :

- dérivé sulfonique de 3-benzylidène 2-bornanone de formule (I) dans laquelle n = 0 (acide benzène 1,4 (di(3-méthylidène campho-10-sulfonique)), neutralisé à 100 % par une polydiméthylsiloxane obtenue par copolymérisation d'un silane difonctionnel et d'un monomère $CH_3$ -Si(OR)$_2$-($CH_2$)$_3$-$NH_2$ où R est éthyle et/ou méthyle (Rhodorsil Huile PL 1300 vendue par Rhône-Poulenc - Indice d'amine 0,92 meq/g).
- dérivé sulfonique de benzophénone de formule (VI) dans lequel a, b et c sont égaux à 0, et R15 désigne un radical méthyle (acide 2-hydroxy 4-méthoxybenzophénone 5-sulfonique : Uvinul MS 40 vendu par BASF), neutralisé à 100 % par le dérivé siliconé aminofonctionnel Rhodorsil Huile PL 1300 cité ci-dessus.

- dérivé sulfonique de benzimidazole de formule (VII) dans laquelle X désigne le radical -NH- et R16 désigne un atome d'hydrogène (acide 2-phénylbenzimidazole 5-sulfonique : Eusolex 232 vendu par MERCK), neutralisé à 100 % par une polydiméthylsiloxane de type AMODIMETHICONE vendue sous l'appellation Silicone Fluid L656 par Wacker Silicone (indice d'amine de 1,2 à 1,4 meq/g).

Le filtre UV hydrophile comportant au moins un radical acide sulfonique est généralement présent dans les compositions filtrantes selon l'invention à une concentration totale comprise entre 0,2 et 10 % environ et de préférence 0,5 et 5 % environ par rapport au poids total de la composition.

Le dérivé siliconé aminofonctionnel est de préférence présent dans les compositions filtrantes en une proportion nécessaire à la neutralisation d'au moins 50 % des fonctions acides sulfoniques du filtre hydrophile.

Encore plus préférentiellement, cette quantité correspond à la neutralisation de 100 % des fonctions acides sulfoniques.

Les compositions cosmétiques selon l'invention peuvent contenir un ou plusieurs filtres solaires complémentaires actifs dans l'UVA et/ou l'UVB, hydrophiles ou lipophiles autres, bien sûr, que les filtres hydrophiles acides de l'invention.

Ces filtres complémentaires sont choisis de préférence parmi les dérivés cinnamiques tels que par exemple le p-méthoxycinnamate de 2-éthylhexyle, les dérivés salicyliques comme par exemple le salicylate de 2-éthylhexyle et le salicylate d'homomenthyle,les dérivés du camphre comme par exemple le 3(4-méthylbenzylidène)camphre, les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de $\beta,\beta$-diphénylacrylate tels que le $\alpha$-cyano-$\beta,\beta$-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665.

Les compositions cosmétiques selon l'invention peuvent encore contenir des nanopigments d'oxydes métalliques enrobés ou non comme par exemple des nanopigments d'oxyde de titane, de fer, de zinc ou de cérium.

Les nanopigments d'oxydes métalliques enrobés ou non enrobés sont en particulier décrits dans la demande EP 0518.772.

Les compositions de l'invention peuvent comprendre en outre des adjuvants cosmétiques choisis parmi les corps gras, les solvants organiques, les épaississants non ioniques, les adoucissants, les antioxydants, les opacifiants, les stabilisants, les silicones autres que celles déjà décrites, les agents anti-mousse, les agents hydratants, les vitamines, les parfums, les conservateurs, les tensioactifs non ioniques, les charges, les séquestrants, les polymères non ioniques, les propulseurs, les agents alcalinisants ou acidifiants, les colorants, ou tout autre ingrédient habituellement utilisé en cosmétique.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en $C_{12}$-$C_{15}$ (Finsolv TN de FINETEX), l'alcool myristique polyoxypropyléné à 3 moles d'oxyde de propylène (WITCONOL APM de WITCO), les triglycérides d'acides gras en $C_6$-$C_{18}$ tels que les triglycérides d'acide caprylique/caprique.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin (octanoate de stéaryle), les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Parmi les solvants organiques, on peut citer les alcools et polyols inférieurs tels que l'éthanol, l'isopropanol, le propylèneglycol, la glycérine et le sorbitol.

Les épaississants de préférence non ioniques, peuvent être choisis parmi les gommes de guar et celluloses modifiées ou non telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

Les compositions de l'invention sont préparées selon les techniques bien connues de l'homme de l'art. Il est possible lors de la préparation de la composition d'empâter le filtre acide avec le dérivé siliconé aminofonctionnel puis d'additionner les autres constituants de la composition et d'homogénéiser. Il est aussi possible de les faire réagir préalablement par exemple par addition d'une solution de dérivé siliconé aminofonctionnel dans l'éthanol ou le dichlorométhane à une solution aqueuse de filtre acide, suivie d'une évaporation du solvant, et d'introduire le produit issu de cette réaction dans la composition.

La composition cosmétique de l'invention peut être utilisée comme composition protectrice de l'épiderme humain

ou des cheveux contre les rayons ultraviolets, comme composition antisolaire ou comme produit de maquillage.

Cette composition peut se présenter en particulier sous forme d'émulsion telle qu'une crème, un lait ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008.

Un autre objet de la présente invention est un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion telle qu'une crème ou un lait, sous forme de pommade, de gel, de bâtonnet solide, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

Un autre objet de l'invention est une association filtrante comprenant d'une part un filtre hydrophile comportant au moins un radical acide sulfonique tel que décrit ci-dessus et, d'autre part, un dérivé siliconé aminofonctionnel tel que décrit également ci-dessus.

Le dérivé siliconé aminofonctionnel est de préférence présent en une proportion nécessaire à la neutralisation d'au moins 50 % des fonctions acides sulfoniques du filtre hydrophile.

Le dérivé siliconé aminofonctionnel est, encore plus préférentiellement, présent en une proportion nécessaire à la neutralisation de 100 % des fonctions acides sulfoniques du filtre hydrophile.

Un autre objet de la présente invention est l'utilisation de l'association ci-dessus pour la préparation de compositions cosmétiques.

L'invention sera plus complètement illustrée par les exemples ci-après qui ne sauraient être considérés comme apportant une quelconque limitation.:

**EXEMPLE 1** : Composition solaire pour la peau (émulsion huile dans eau)

| | |
|---|---|
| Mélange (80/20) d'alcool cétylstéarylique et d'alcool cétylstéarylique oxyéthyléné à 33 moles d'oxyde d'éthylène vendu sous la dénomination SINNOWAX AO par la Société Henkel | 7 g |
| Stéarate de glycérol | 2 g |
| Alcool cétylique | 1,5 g |
| Polydiméthylsiloxane : silicone DC200-350 cst de la Société Dow Corning | 1,5 g |
| p-méthoxycinnamate d'octyle | 4 g |
| 2-cyano, 3,3-diphényl 2-propénoate d'octyle | 10 g |
| Microtitanium Dioxyde MT 100T de Tayca | 2 g |
| Silicone Fluid L650 de Wacker Silicone d'indice d'amine 2,7 à 3,2 meq/g | 1,18 g |
| Acide benzène 1,4 [di(3-méthylidènecampho-10-sulfonique)] | 1 g |
| Glycérine | 20 g |
| Conservateurs qs | |

(suite)

| | | |
|---|---|---|
| Eau déminéralisée | qsp | 100 g |

On réalise l'émulsion en additionnant la phase grasse portée aux environs de 80°C à la phase aqueuse renfermant les conservateurs et la glycérine portée à la même température et sous agitation rapide.

**EXEMPLE 2**

Composition solaire pour la peau : émulsion huile dans eau :

| | |
|---|---|
| Mélange de stéarate de glycérol et de stéarate de polyéthylèneglycol à 100 moles d'oxyde d'éthylène vendu sous la dénomination ARLACEL 165 par la Société ICI | 2 g |
| Stéarate de glycérol | 2 g |
| Alcool stéarylique | 1 g |
| 2-cyano, 3,3-diphényl 2-propénoate d'octyle | 7 g |
| Acide 2-hydroxy 4-méthoxybenzophénone 5-sulfonique vendu sous la dénomination UVINUL MS 40 par la société BASF | 2 g |
| Silicone Fluid L655 de WACKER SILICONE d'indice d'amine 1,3 à 1,45 meq/g | 5 g |
| Cétylhydroxyéthylcellulose (Natrosol Plus Grade 330 CS d'AQUALON) | 0,4 g |
| Glycérine | 9 g |
| Conservateurs qs | |
| Eau déminéralisée qsp | 100 g |

Cette émulsion est réalisée selon le procédé décrit à l'exemple 1, la phase aqueuse contenant l'épaississant (cetylhydroxyéthylcellulose), les conservateurs et la glycérine.

**EXEMPLE 3**

Composition solaire pour la peau : émulsion huile dans eau :

| | |
|---|---|
| Mélange de stéarate de glycérol et de stéarate de polyéthylèneglycol à 100 moles d'oxyde d'éthylène vendu sous la dénomination ARLACEL 165 par la Société ICI | 2g |
| Stéarate de glycérol | 2 g |
| Alcool stéarylique | 1 g |
| p-méthoxycinnamate d'octyle | 5 g |
| Acide 2-phényl benzimidazole 5-sulfonique vendu sous la dénomination EUSOLEX 232 par la société MERCK | 4 g |
| Silicone Fluid L656 de WACKER SILICONE d'indice d'amine 1,2 à 1,4 meq/g | 11,23 g |
| Cétylhydroxyéthylcellulose (Natrosol Plus Grade 330 CS d'AQUALON) | 0,4 g |
| Glycérine | 9 g |
| Conservateurs qs | |
| Eau déminéralisée qsp | 100 g |

Cette émulsion est réalisée suivant le procédé décrit à l'exemple 2.

**EXEMPLE 4**

Composition solaire pour la peau : émulsion eau dans huile :

| | |
|---|---|
| ARLACEL 1689 d'ICI (mélange de ricinoléate de polyglycérol (3 moles) et d'oléate de Sorbitol) | 3,5 g |
| Huile de vaseline | 10 g |
| Copolymère bloc Dodecylglycol/Ethylèneglycol : Elfacos ST37 d'AKZO (stabilisant) | 1 g |
| Polydiméthylsiloxane : Silicone DC 200-350 cst de DOW CORNING | 3g |
| 12-Hydroxystéarate de β-hydroxyoctacosanyle : Elfacos C26 d'AKZO (stabilisant) | 5 g |
| Acide benzène 1,4 [di(3-méthylidènecampho-10-sulfonique)] | 2g |
| Silicone Fluid L650 de WACKER SILICONE d'indice d'amine 2,7 à 3,2 meq/g | 2,37 g |
| Glycérine | 3 g |
| Conservateurs qs | |
| Mélange de diméthiconol (13 %) et d'octaméthylcyclotétrasiloxane - décaméthylcyclopentasiloxane (87 %) vendu sous la dénomination Q2-1401 par la société DOW CORNING | 5 g |
| Eau déminéralisée qsp | 100 g |

On réalise cette émulsion en additionnant la phase aqueuse renfermant les conservateurs et la glycérine portée aux environs de 80° C à la phase grasse portée à la même température sous agitation vive.

**EXEMPLE 5**

Composition solaire pour la peau.

Emulsion huile dans eau dont la phase aqueuse contient une dispersion vésiculaire non-ionique.

On prépare au préalable une dispersion vésiculaire non-ionique de la façon suivante : on forme une phase lamellaire en ajoutant une quantité d'eau chaude (environ 80° C) (15,2 g) équivalente à 2 fois la quantité de lipides fondus suivants :

Liquide non ionique de formule :

$C_{12}H_{25}-[OC_2H_3(R)-]-O-[-C_3H_5\text{-}(OH)\text{-}O-]_n-H$ 6 g

dans laquelle :

. $-C_3H_5(OH)O-$ est constitué par un mélange de radicaux :

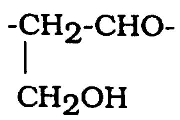

et

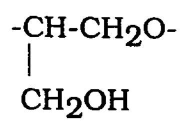

. -O $C_2H_3$ (R) - est constitué par un mélange de radicaux :

$$- O - CHR - CH_2 - \text{et} - O - CH_2 - CHR -$$

.n est une valeur statistique moyenne égale à 6
.R est un mélange de radicaux $C_{14}H_{29}$ et $C_{16}H_{33}$.

Cholesterol 1,6 g

Après agitation quelques minutes à l'Ultra-Turrax et retour à la température ambiante, on lui ajoute pour foyer la phase vésiculaire, une quantité d'eau à température ambiante (22,8 g) équivalente à 3 fois la quantité de lipides précédents et renfermant 5 g de glycérine puis on agite quelques minutes à l'Ultra Turrax.

On empâte ensuite 2 g d'acide benzène 1,4 [di(3-méthylidènecampho-10-sulfonique)] par 7,68g de Rhodorsil Huile PL 1300 vendu par la société Rhône-Poulenc, d'indice d'amine 0,92 meq/g, auxquels on additionne les autres composants de la phase grasse : 15 g d'huile de vaseline et des conservateurs et l'on porte à 70° C. Après retour à température ambiante, on émulsionne ensuite cette phase grasse par la dispersion vésiculaire préparée ci-dessus puis on épaissit en final la composition en lui ajoutant 0,2 g de cétylhydroxyéthylcellulose (Natrosol Plus Grade 330 CS d'Aqualon) et 0,2 g de gomme de guar hydroxypropylée et des conservateurs dissous dans le restant d'eau nécessaire à porter la composition à 100 g.

**EXEMPLE 6**

Crème de soin pour la peau

On opère comme à l'exemple 5 mais en utilisant 3 g d'acide 1,4 [di(3-méthylidènecampho-10-sulfonique)] et 5 g de polydiméthylsiloxane à groupements terminaux amine primaire vendu par la société Goldschmidt sous la dénomination Tegomer A-SI-2120, d'indice d'amine : 1,95 meq/g en remplacement de la Rhodorsil Huile PL 1300 de Rhône-Poulenc.

**EXEMPLE 7**

Après shampooing

| | |
|---|---|
| Alcool cétyl stéarylique 30/70 oxyéthyléné 33 OE | 1 g |
| Alcool cétyl stéarylique 30/70 | 4,5 g |
| 2-octyl dodécanol | 0,8 g |
| Glycérine | 0,8 g |
| Alcool cétyl stéarylique 50/50 | 2 g |
| Acide benzène 1,4 [di(3-méthyildènecampho-10-sulfonique)] | 2 g |
| Silicone Fluid L650 de WACKER silicone d'indice d'amine 2,7 à 3,2 meq/g | 2,36 g |
| Conservateurs qs | |
| Parfum qs | |
| Eau purifiée qsp | 100 g |

Cet après-shampoing est préparé selon le procédé de l'exemple 1.

**EXEMPLE 8**

Composition solaire pour la peau : émulsion huile-dans-eau

| | |
|---|---|
| Mélange de stéarate de glycérol et de stéarate de polyéthylèneglycol à 100 moles d'oxyde d'éthylène vendu sous la dénomination ARLACEL 165 par la société ICI | 2 g |
| Huile de vaseline | 10 g |
| Stéarate de glycérol | 2 g |
| Alcool stéarylique | 1 g |
| Glycérine | 9 g |
| Acide benzène 1,4 (di(3-méthylidène campho-10-sulfonique)) | 3 g |
| Rhodorsil Huile PL 1300 vendue par Rhône-Poulenc - Indice d'amine 0,92 meq/g | 11,52 g |
| Cétylhydroxyéthylcellulose (Natrosol Plus Grade 330 CS d'Aqualon) | 0,4 g |
| Conservateurs qs | |
| Eau qsp | 100 g |

Cette émulsion est préparée selon le procédé décrit à l'exemple 2

**EXEMPLE 9**

Composition solaire pour la peau : émulsion huile-dans-eau

| | |
|---|---|
| Mélange de stéarate de glycérol et de stéarate de polyéthylèneglycol à 100 moles d'oxyde d'éthylène vendu sous la dénomination ARLACEL 165 par la société ICI | 2 g |
| Huile de vaseline | 10 g |
| Stéarate de glycérol | 2 g |
| Alcool stéarylique | 1 g |
| Glycérine | 9 g |
| Acide 2-hydroxy 4-méthoxybenzophénone 5-sulfonique (Uvinul MS 40 de BASF) | 2g |
| Rhodorsil Huile PL 1300 vendue par Rhône-Poulenc - Indice d'amine 0,92 meq/g | 7,06 g |
| Cétylhydroxyéthylcellulose (Natrosol Plus Grade 330 CS d'Aqualon) | 0,4 g |
| Conservateurs qs | |
| Eau qsp | 100 g |

Cette émulsion est préparée selon le procédé décrit à l'exemple 2.

**EXEMPLE 10**

Composition solaire pour la peau : émulsion huile-dans-eau

| | |
|---|---|
| Mélange de stéarate de glycérol et de stéarate de polyéthylèneglycol à 100 moles d'oxyde d'éthylène vendu sous la dénomination ARLACEL 165 par la société ICI | 2 g |
| Huile de vaseline | 10 g |
| Stéarate de glycérol | 2 g |
| Alcool stéarylique | 1 g |
| Glycérine | 9 g |
| Acide 2-phényl-benzimidazole 5-sulfonique : Eusolex 232 de MERCK | 4 g |
| Silicone Fluid L 656 vendue par WACKER SILICONE - Indice d'amine 1, 2 à 1,4 meq/g | 11,23 g |
| Cétylhydroxyéthylcellulose : (Natrosol Plus Grade 330 CS d'Aqualon) | 0,4 g |
| Conservateurs qs | |
| Eau qsp | 100 g |

Cette émulsion est préparée selon le procédé décrit dans l'exemple 2

**Revendications**

1. Composition cosmétique filtrante, caractérisée en ce qu'elle contient, dans un support cosmétiquement acceptable, au moins un agent hydrophile filtrant le rayonnement ultraviolet comportant au moins un radical acide sulfonique $-SO_3H$ et au moins un dérivé siliconé amino fonctionnel.

2. Composition selon la revendication 1, caractérisée en ce que l'agent hydrophile filtrant le rayonnement ultraviolet est un composé de structure 3-benzylidène 2-bornanone.

3. Composition selon la revendication 2, caractérisé en ce que l'agent hydrophile filtrant le rayonnement ultraviolet répond à la formule (I) suivante :

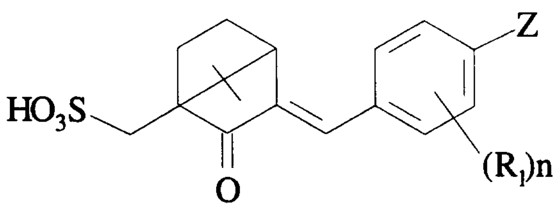

(I)

dans laquelle :

- Z désigne un groupement de formule :

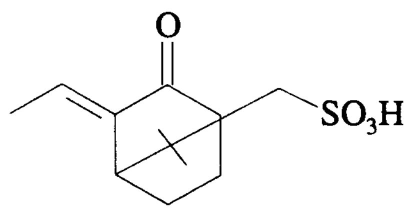

- n désigne 0 ou un nombre entier supérieur ou égal à 1 et inférieur ou égal à 4
- $R_1$ représente un ou plusieurs radicaux alkyle ou alkoxy, identiques ou différents, linéaires ou ramifiés, contenant de 1 à 4 atomes de carbone.

**4.** Composition selon la revendication 3, caractérisée en ce que le composé de formule (I) est l'acide benzène 1,4 [di(3-méthylidènecampho-10-sulfonique)].

**5.** Composition selon la revendication 2, caractérisé en ce que l'agent hydrophile filtrant le rayonnement ultraviolet répond à la formule (II) suivante :

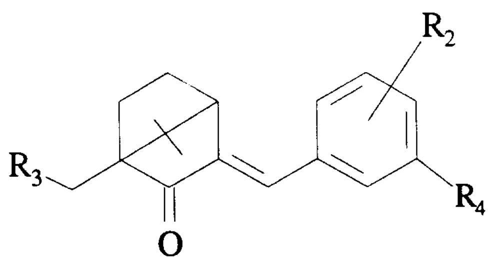

(II)

dans laquelle

- $R_2$ désigne un atome d'hydrogène, un atome d'halogène, un radical alkyle contenant 1 à 4 atomes de carbone, un radical $-SO_3H$

- $R_3$ et $R_4$ désignent un atome d'hydrogène ou un radical $-SO_3H$, au moins un des radicaux $R_2$, $R_3$ ou $R_4$ désignant le radical $-SO_3H$, $R_2$ et $R_4$ ne pouvant désigner simultanément un radical-$SO_3H$.

**6.** Composition selon la revendication 5, caractérisée en ce que dans la formule (II) $R_2$ désigne le radical $-SO_3H$ en position para du benzylidène camphre et $R_3$ et $R_4$ représentent chacun un atome d'hydrogène.

**7.** Composition selon la revendication 5, caractérisée en ce que dans la formule (II) $R_2$ et $R_4$ représente chacun un atome d'hydrogène et $R_3$ désigne $-SO_3H$.

**8.** Composition selon la revendication 5, caractérisée en ce que dans la formule (II) $R_2$ désigne le radical méthyle en position para du benzylidène camphre, $R_4$ un radical représente le radical $SO_3H$ et $R_3$ un atome d'hydrogène.

**9.** Composition selon la revendication 5, caractérisée en ce que dans la formule (II) $R_2$ désigne un atome de chlore en position para du benzylidène camphre, $R_4$ le radical $-SO_3H$ et $R_3$ un atome d'hydrogène.

**10.** Composition selon la revendication 5, caractérisée en ce que dans la formule (II) $R_2$ désigne le radical méthyle en position para du benzylidène camphre, $R_4$ désigne un atome d'hydrogène et $R_3$ désigne le radical $-SO_3H$.

**11.** Composition selon la revendication 2, caractérisée en ce que l'agent hydrophile filtrant le rayonnement ultraviolet répond à la formule (III) suivante :

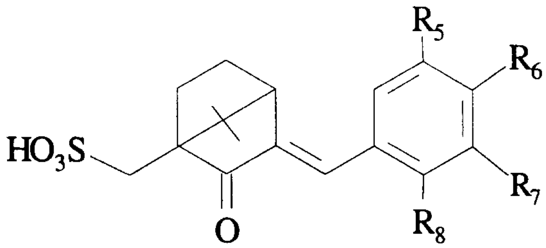

(III)

dans laquelle :

- $R_5$ et $R_7$, identiques ou différents, désignent un atome d'hydrogène, un radical hydroxyle, un radical alkyle linéaire ou ramifié contenant 1 à 8 atomes de carbone ou un radical alcoxy linéaire ou ramifié contenant 1 à 8 atomes de carbone, l'un au moins des radicaux $R_5$ et $R_7$ représentant un radical hydroxyle, alkyle ou alcoxy,
- $R_6$ et $R_8$, identiques ou différents, désignent un atome d'hydrogène, un radical hydroxyle, l'un au moins des radicaux $R_6$ et $R_8$ désignant le radical hydroxyle,
- sous réserve que lorsque $R_5$ et $R_8$ désignent l'hydrogène et que $R_6$ désigne le radical hydroxyle, $R_7$ ne désigne pas un radical alcoxy ou un atome d'hydrogène.

**12.** Composition selon la revendication 11, caractérisée en ce que dans la formule (III) $R_5$ est un radical méthyle, $R_6$ un atome d'hydrogène, $R_7$ un radical tertiobutyle et $R_8$ un radical hydroxyle.

**13.** Composition selon la revendication 11, caractérisée en ce que dans la formule (III) $R_5$ est un radical méthoxy, $R_6$ un atome d'hydrogène, $R_7$ un radical tertiobutyle et $R_8$ un radical hydroxyle.

**14.** Composition selon la revendication 11, caractérisée en ce que dans la formule (III) $R_5$ et $R_7$ désignent chacun un radical tertiobutyle, $R_6$ un radical hydroxyle et $R_8$ un atome d'hydrogène.

**15.** Composition selon la revendication 2, caractérisée en ce que l'agent hydrophile filtrant le rayonnement ultraviolet répond à la formule (IV) suivante :

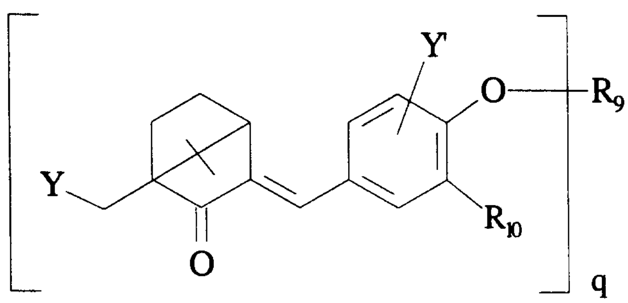

(IV)

dans laquelle

- $R_9$ désigne un atome d'hydrogène, un radical alkyle linéaire ou ramifié contenant de 1 à 18 atomes de carbone , un radical alcényle, linéaire ou ramifié, contenant de 3 à 18 atomes de carbone, un groupement choisi parmi le groupe comprenant :

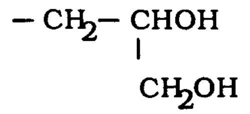

,

$-(CH_2CH_2O)_n-H$, ou $-CH_2-CHOH-CH_3$,
ou un radical divalent : $-(CH_2)_m-$ ou $-CH_2-CHOH-CH_2-$
n étant un nombre entier compris entre 1 et 6 ($1 \leq n \leq 6$) et m un nombre entier compris entre 1 et 10 ($1 \leq m \leq 10$).

- $R_{10}$ désigne un atome d'hydrogène, un radical alcoxy contenant de 1 à 4 atomes de carbone, ou un radical divalent - O - relié au radical $R_9$ lorsque celui-ci est divalent lui aussi.

- q désigne un nombre entier égal à 1 ou 2, étant entendu que si q est égal à 2, $R_9$ doit désigner un radical divalent.

- Y et Y' désignent un atome d'hydrogène ou un radical $-SO_3H$, au moins un de ces radicaux Y ou Y, est différent de l'hydrogène.

**16.** Composition selon la revendication 15, caractérisée en ce que dans la formule (IV) q est égal à 1, Y et $R_{10}$ désignent chacun un atome d'hydrogène, $R_9$ désigne un radical méthyle, Y' en position 3 désigne un radical $-SO_3H$.

**17.** Composition selon la revendication 15, caractérisée en ce que dans la formule (IV) q est égal à 1, Y désigne un radical $-SO_3H$, Y' un atome d'hydrogène; $R_{10}$ un radical divalent -O- relié à $R_9$ désignant un radical méthylène.

**18.** Composition selon la revendication 15, caractérisée en ce que dans la formule (IV) q est égal à 1, Y désigne un radical $-SO_3H$, Y' et $R_{10}$ désignent tous deux un atome d'hydrogène; $R_9$ désigne un radical méthyle.

**19.** Composition selon la revendication 15, caractérisée en ce que dans la formule (IV) q est égal à 1, Y désigne un radical $-SO_3H$, Y' un atome d'hydrogène; $R_9$ désigne un radical méthyle, $R_{10}$ désigne un radical méthoxy.

**20.** Composition selon la revendication 15, caractérisée en ce que dans la formule (IV) q est égal à 1, Y désigne un radical $-SO_3H$, Y' et $R_{10}$ désignent tous deux un atome d'hydrogène; $R_9$ un radical n butyle.

**21.** Composition selon la revendication 15, caractérisée en ce que dans la formule (IV) q est égal à 1, Y désigne un radical $-SO_3H$, Y' un atome d'hydrogène; $R_9$ désigne un radical n butyle, $R_{10}$ un radical méthoxy.

**22.** Composition selon la revendication 2, caractérisée en ce que l'agent hydrophile filtrant le rayonnement ultraviolet répond à la formule (V) suivante :

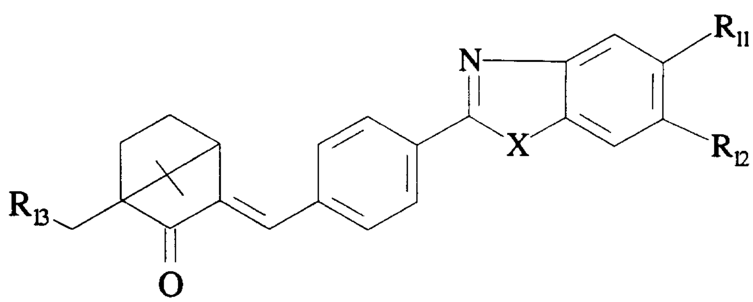

(V)

dans laquelle :

- $R_{11}$ désigne un atome d'hydrogène, un radical alkyle ou alcoxy, linéaire ou ramifié, contenant 1 à 6 atomes de

carbone, un radical $-SO_3H$.

- $R_{12}$ désigne un atome d'hydrogène, un radical alkyle ou alcoxy linéaire ou ramifié contenant 1 à 6 atomes de carbone.
- $R_{13}$ désigne un atome d'hydrogène, ou un radical $-SO_3H$.
- l'un au moins des radicaux $R_{11}$ ou $R_{13}$ désigne un radical $-SO_3H$.
- X est un atome d'oxygène ou de soufre, ou un groupement -NR-, R étant un atome d'hydrogène ou un radical alkyle linéaire ou ramifié contenant de 1 à 6 atomes de carbone.

**23.** Composition selon la revendication 22, caractérisée en ce que dans la formule (V) X désigne un radical -NH-, $R_{11}$désigne un radical $-SO_3H$, $R_{12}$ et $R_{13}$ désignent tous deux un atome d'hydrogène.

**24.** Composition selon la revendication 1, caractérisée en ce que l'agent hydrophile filtrant le rayonnement ultraviolet est un composé de formule (VI)

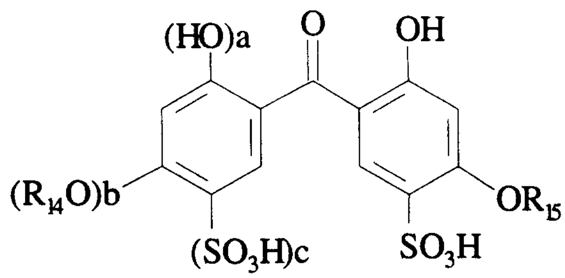

(VI)

dans laquelle

- $R_{14}$ et $R_{15}$ identiques ou différents, désignent un atome d'hydrogène ou un radical alkyle, linéaire ou ramifié, contenant de 1 à 8 atomes de carbone - a, b et c identiques ou différents sont égaux à 0 ou 1.

**25.** Composition selon la revendication 24, caractérisée en ce que dans la formule (VI) a=b=c=0 et $R_{15}$ désigne un radical méthyle.

**26.** Composition selon la revendication 1, caractérisée par le fait que l'agent hydrophile filtrant le rayonnement ultraviolet est un composé de formule (VII)

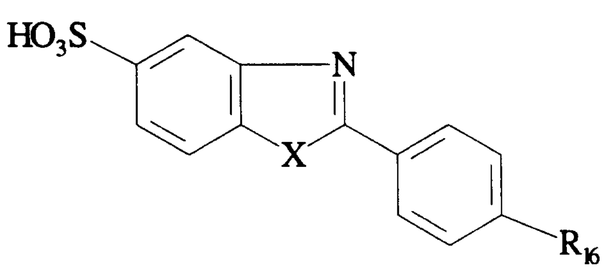

(VII)

dans laquelle

- X désigne un atome d'oxygène ou un radical - NH -
- $R_{16}$ désigne un atome d'hydrogène, un radical alkyle ou alcoxy, linéaire ou ramifié, contenant de 1 à 8 atomes de carbone ou un groupement de formule (VIII)

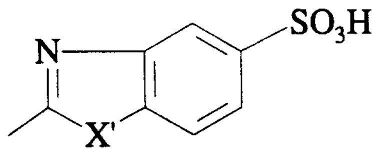

(VIII)

dans laquelle X' désigne indépendamment de X un atome oxygène ou un radical - NH -

**27.** Composition selon la revendication 26, caractérisée en ce que dans la formule (VII) X désigne le radical -NH-, et $R_{16}$ désigne un atome d'hydrogène.

**28.** Composition selon la revendication 26, caractérisée en ce que dans la formule (VII) X désigne le radical -NH-, $R_{16}$ désigne le groupement de formule (VIII) avec X' désignant le radical -NH-.

**29.** Composition selon la revendication 26, caractérisée en ce que dans la formule (VII) X désigne un atome d'oxygène, $R_{16}$ désigne le groupement de formule (VIII) avec X' désignant un atome d'oxygène.

**30.** Composition selon l'une quelconque des revendications 1 à 29, caractérisée en ce que le dérivé siliconé aminofonctionnel présente un indice d'amine supérieur à 0,25 meq/gramme.

**31.** Composition selon la revendication 30, caractérisée en ce que le dérivé siliconé aminofonctionnel présente un indice d'amine supérieur à 0,50 meq/gramme.

**32.** Composition selon l'une quelconque des revendications 1 à 31, caractérisée en ce que le dérivé siliconé aminofonctionnel est un dérivé siliconé comportant une fonction amine primaire, secondaire ou tertiaire.

**33.** Composition selon la revendication 32, caractérisée par le fait que le dérivé siliconé comporte une fonction amine primaire $NH_2$ reliée à la chaîne principale par l'intermédiaire d'un groupement pendant, ou en position $\alpha$ et $\omega$ sur la chaîne principale.

**34.** Composition selon la revendication 32, caractérisée en ce que le dérivé siliconé comportant une fonction amine secondaire est un composé aminobispropyldiméthicone.

**35.** Composition selon la revendication 32, caractérisée en ce que le dérivé siliconé comportant une fonction amine tertiaire est le composé de structure

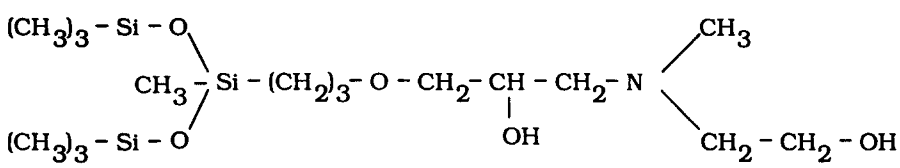

**36.** Composition selon l'une quelconque des revendications précédentes, caractérisée par le fait qu'elle renferme 0,2 à 10 % en poids d' agent hydrophile filtrant le rayonnement ultraviolet et de préférence 0,5 à 5 %.

**37.** Composition selon l'une quelconque des revendications précédentes, caractérisée par le fait qu'elle renferme le dérivé siliconé aminofonctionnel en une proportion nécessaire à la neutralisation d'au moins 50 % des fonctions acides sulfoniques du filtre hydrophile.

**38.** Composition selon l'une quelconque des revendications précédentes, caractérisée en ce qu'elle renferme le dérivé siliconé aminofonctionnel en une proportion nécessaire à la neutralisation de 100 % des fonctions acides sulfoniques du filtre hydrophile.

**39.** Composition selon l'une quelconque des revendications précédentes, caractérisée par le fait qu'elle contient en outre un ou plusieurs filtres solaires complémentaires UVB et/ou UVA hydrophiles ou lipophiles autres que les filtres hydrophiles acides selon la revendication 1.

**40.** Composition selon la revendication 39, caractérisée par le fait que les filtres solaires complémentaires sont choisis parmi les cinnamates, les salicylates, les dérivés du benzylidène camphre, les dérivés de triazine, les dérivés de la benzophénone, les dérivés du dibenzoylméthane, les dérivés de β,β-diphénylacrylate, les dérivés de l'acide p-aminobenzoïque, l'anthranilate de menthyle, les polymères filtres et les silicones filtres.

**41.** Composition selon la revendication 40, caractérisée par le fait que les filtres solaires complémentaires sont choisis parmi le groupe comprenant le 4-tert-butyl-4'-méthoxydibenzoylméthane, le 2-cyano 3,3-diphényl 2-propénoate d'octyle et le 3-(4-méthylbenzylidène)-camphre.

**42.** Composition selon la revendication 40, caractérisée par le fait que les filtres solaires complémentaires sont choisis parmi le groupe comprenant le p-méthoxycinnamate de 2-éthylhexyle, le 2,4,6-tris [ p-(2'éthylhexyl-1'-oxycarbonyl)anilino] 1,3,5-triazine et le 2-cyano 3,3-diphényl 2-propénoate d'octyle.

**43.** Composition selon l'une quelconque des revendications précédentes, caractérisée en ce qu'elle contient en outre des nanopigments d'oxydes métalliques enrobés ou non.

**44.** Composition selon l'une quelconque des revendications précédentes, caractérisée par le fait qu'elle comprend en outre des adjuvants cosmétiques choisis parmi les corps gras, les solvants organiques, les épaississants non ioniques, les adoucissants, les antioxydants, les opacifiants, les stabilisants, les silicones autres que les dérivés siliconés aminofonctionnels, les agents anti-mousse, les agents hydratants, les parfums, les conservateurs, les tensioactifs non ioniques, les charges, les séquestrants, les polymères non ioniques, les propulseurs, les agents alcalinisants ou acidifiants, les colorants.

**45.** Composition selon l'une quelconque des revendications précédentes, caractérisée en ce qu'elle constitue une composition protectice de l'épiderme humain ou une composition antisolaire et se présente sous forme de dispersion vésiculaire non ionique, émulsions, crème, lait, gel, gel crème, suspensions, dispersions, poudre, bâtonnet solide, mousse ou spray.

**46.** Composition selon l'une quelconque des revendications 1 à 44, caractérisée en ce qu' elle constitue une composition de maquillage des cils, des sourcils ou de la peau et se présente sous forme solide ou pâteuse, anhydre ou aqueuse, d'émulsion, de suspension ou de dispersion.

**47.** Composition selon l'une quelconque des revendications 1 à 44 utilisée pour la protection des cheveux contre les rayons ultraviolets, caractérisée par le fait qu'elle se présente sous forme de shampooing, de lotion, de gel, d'émulsion, de dispersion vésiculaire non ionique ou de laque pour cheveux.

**48.** Composition selon l'une quelconque des revendications précédentes, caractérisée en ce que ledit support cosmétiquement acceptable est une émulsion de type huile-dans-eau.

**49.** Procédé de traitement cosmétique de la peau et/ou des cheveux pour les protéger contre les effets des rayons UV de longueurs d'onde comprises entre 280 et 400 nm, caractérisé par le fait qu'il consiste à appliquer une quantité efficace d'une composition cosmétique filtrante substantive telle que définie dans l'une quelconque des revendications 1 à 48.

**50.** Association filtrante comprenant d'une part un filtre hydrophile comportant au moins un radical acide sulfonique selon l'une quelconque des revendications 1 à 29 et d'autre part un dérivé siliconé aminofonctionnel selon l'une quelconque des revendications 30 à 35.

**51.** Association selon la revendication 50, caractérisée en ce que le dérivé siliconé aminofonctionnel est présent en une proportion nécessaire à la neutralisation d'au moins 50 % des fonctions acides sulfoniques du filtre hydrophile.

**52.** Association selon la revendication 50 ou 51, caractérisée en ce que le dérivé siliconé aminofonctionnel est présent

en une proportion nécessaire à la neutralisation de 100 % des fonctions acides sulfoniques du filtre hydrophile.

**53.** Utilisation de l'association selon l'une quelconque des revendications 50 à 52 pour la préparation de compositions cosmétiques.

## Claims

**1.** Screening cosmetic composition, characterized in that it contains, in a cosmetically acceptable vehicle, at least one hydrophilic agent which screens ultraviolet radiation, comprising at least one sulphonic acid radical $-SO_3H$ and at least amino-functional silicone derivative.

**2.** Composition according to Claim 1, characterized in that the hydrophilic agent which screens ultraviolet radiation is a compound with a 3-benzylidene-2-bornanone structure.

**3.** Composition according to Claim 2, characterized in that the hydrophilic agent which screens ultraviolet radiation corresponds to the following formula (I):

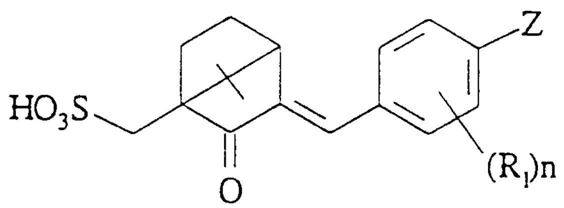

(I)

in which:

- Z denotes a group of formula:

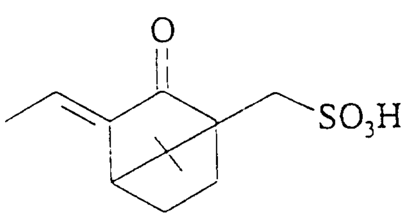

- n denotes 0 or an integer greater than or equal to 1 and less than or equal to 4
- $R_1$ represents one or more identical or different, linear or branched, alkyl or alkoxy radicals containing 1 to 4 carbon atoms.

**4.** Composition according to Claim 3, characterized in that the compound of formula (I) is benzene-1,4-[di(3-methylidenecamphor-10-sulphonic)] acid.

**5.** Composition according to Claim 2, characterized in that the hydrophilic agent which screens ultraviolet radiation corresponds to the following formula (II):

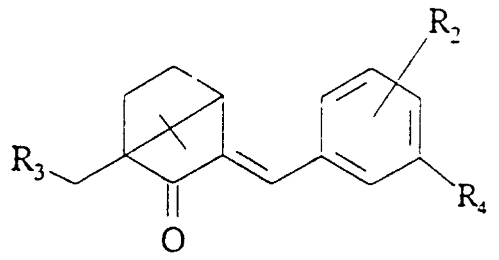

(II)

in which

- $R_2$ denotes a hydrogen atom, a halogen atom, an alkyl radical containing 1 to 4 carbon atoms or a -$SO_3H$ radical
- $R_3$ and $R_4$ denote a hydrogen atom or a -$SO_3H$ radical, at least one of the radicals $R_2$, $R_3$ or $R_4$ denoting the -$SO_3H$ radical and $R_2$ and $R_4$ being unable simultaneously to denote a -$SO_3H$ radical.

**6.** Composition according to Claim 5, characterized in that, in the formula (II), $R_2$ denotes the -$SO_3H$ radical in the para position of the benzylidenecamphor and $R_3$ and $R_4$ each represent a hydrogen atom.

**7.** Composition according to Claim 5, characterized in that, in the formula (II), $R_2$ and $R_4$ each represent a hydrogen atom and $R_3$ denotes -$SO_3H$.

**8.** Composition according to Claim 5, characterized in that, in the formula (II), $R_2$ denotes the methyl radical in the para position of the benzylidenecamphor, $R_4$ represents the radical $SO_3H$ and $R_3$ represents a hydrogen atom.

**9.** Composition according to Claim 5, characterized in that, in the formula (II), $R_2$ denotes a chlorine atom in the para position of the benzylidenecamphor, $R_4$ denotes the -$SO_3H$ radical and $R_3$ denotes a hydrogen atom.

**10.** Composition according to Claim 5, characterized in that, in the formula (II), $R_2$ denotes the methyl radical in the para position of the benzylidenecamphor, $R_4$ denotes a hydrogen atom and $R_3$ denotes the -$SO_3H$ radical.

**11.** Composition according to Claim 2, characterized in that the hydrophilic agent which screens ultraviolet radiation corresponds to the following formula (III):

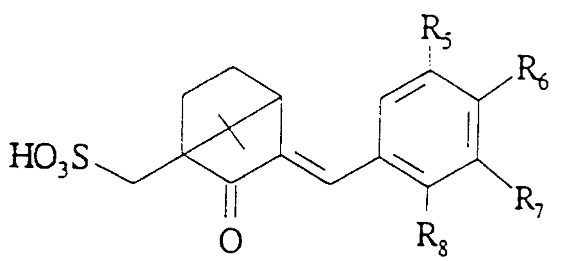

(III)

in which:

- $R_5$ and $R_7$, which are identical or different, denote a hydrogen atom, a hydroxyl radical, a linear or branched alkyl radical containing 1 to 8 carbon atoms or a linear or branched alkoxy radical containing 1 to 8 carbon atoms, at least one of the radicals $R_5$ and $R_7$ representing a hydroxyl, alkyl or alkoxy radical,
- $R_6$ and $R_8$, which are identical or different, denote a hydrogen atom or a hydroxyl radical, at least one of the radicals $R_6$ and $R_8$ denoting the hydroxyl radical,
- with the proviso that, when $R_5$ and $R_8$ denote hydrogen and $R_6$ denotes the hydroxyl radical, $R_7$ does not denote an alkoxy radical or a hydrogen atom.

**12.** Composition according to Claim 11, characterized in that, in the formula (III), $R_5$ is a methyl radical, $R_6$ is a hydrogen atom, $R_7$ is a tert-butyl radical and $R_8$ is a hydroxyl radical.

**13.** Composition according to Claim 11, characterized in that, in the formula (III), $R_5$ is a methoxy radical, $R_6$ is a hydrogen atom, $R_7$ is a tert-butyl radical and $R_8$ is a hydroxyl radical.

**14.** Composition according to Claim 11, characterized in that, in the formula (III), $R_5$ and $R_7$ each denote a tert-butyl radical, $R_6$ denotes a hydroxyl radical and $R_8$ denotes a hydrogen atom.

**15.** Composition according to Claim 2, characterized in that the hydrophilic agent which screens ultraviolet radiation corresponds to the following formula (IV):

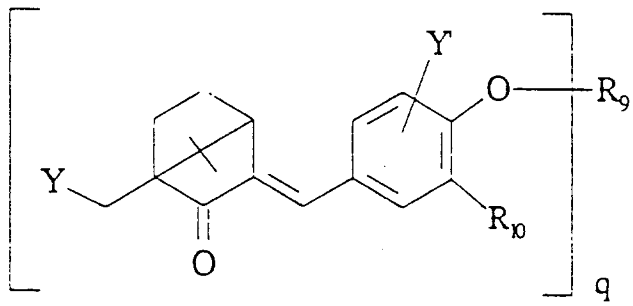

(IV)

in which

- $R_9$ denotes a hydrogen atom, a linear or branched alkyl radical containing 1 to 18 carbon atoms, a linear or branched alkenyl radical containing 3 to 18 carbon atoms, a group chosen from the group comprising:

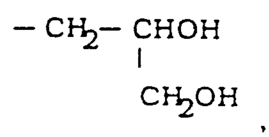

  $-(CH_2CH_2O)_n-H$ or $-CH_2-CHOH-CH_3$,
  or a divalent radical: $-(CH_2)_m-$ or $-CH_2-CHOH-CH_2-$
  n being an integer between 1 and 6 ($1 \leq n \leq 6$) and m being an integer between 1 and 10 ($1 \leq m \leq 10$).
- $R_{10}$ denotes a hydrogen atom, an alkoxy radical containing 1 to 4 carbon atoms or a divalent radical -O-which is linked to the radical $R_9$ where the latter is also divalent.
- q denotes an integer equal to 1 or 2, it being understood that, if q is equal to 2, $R_9$ must denote a divalent radical.
- Y and Y' denote a hydrogen atom or a $-SO_3H$ radical, and at least one of these radicals Y or Y' is different from hydrogen.

**16.** Composition according to Claim 15, characterized in that, in the formula (IV), q is equal to 1, Y and $R_{10}$ each denote a hydrogen atom, $R_9$ denotes a methyl radical and Y' in position 3 denotes a $-SO_3H$ radical.

**17.** Composition according to Claim 15, characterized in that, in the formula (IV), q is equal to 1, Y denotes a $-SO_3H$ radical, Y' denotes a hydrogen atom; $R_{10}$ denotes a divalent radical -O- which is linked to $R_9$ denoting a methylene radical.

**18.** Composition according to Claim 15, characterized in that, in the formula (IV), q is equal to 1, Y denotes a $-SO_3H$ radical, both Y' and $R_{10}$ denote a hydrogen atom; $R_9$ denotes a methyl radical.

**19.** Composition according to Claim 15, characterized in that, in the formula (IV), q is equal to 1, Y denotes a $-SO_3H$

radical, Y' denotes a hydrogen atom; $R_9$ denotes a methyl radical and $R_{10}$ denotes a methoxy radical.

**20.** Composition according to Claim 15, characterized in that, in the formula (IV), q is equal to 1, Y denotes a $-SO_3H$ radical, both Y' and $R_{10}$ denote a hydrogen atom; $R_9$ denotes an n-butyl radical.

**21.** Composition according to Claim 15, characterized in that, in the formula (IV), q is equal to 1, Y denotes a $-SO_3H$ radical, Y' denotes a hydrogen atom; $R_9$ denotes an n-butyl radical and $R_{10}$ denotes a methoxy radical.

**22.** Composition according to Claim 2, characterized in that the hydrophilic agent which screens ultraviolet radiation corresponds to the following formula (V):

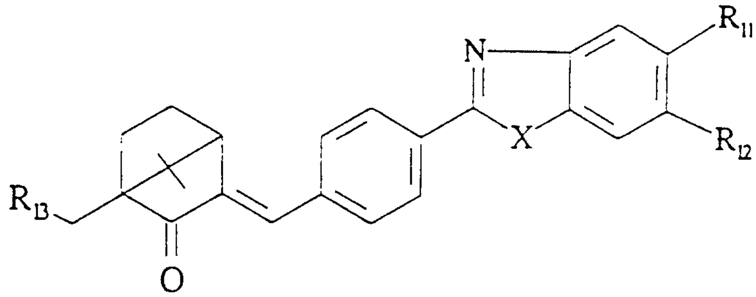

(V)

in which:

- $R_{11}$ denotes a hydrogen atom, a linear or branched, alkyl or alkoxy radical containing 1 to 6 carbon atoms or a $-SO_3H$ radical.
- $R_{12}$ denotes a hydrogen atom or a linear or branched, alkyl or alkoxy radical containing 1 to 6 carbon atoms.
- $R_{13}$ denotes a hydrogen atom or a $-SO_3H$ radical.
- at least one of the radicals $R_{11}$ or $R_{13}$ denotes a $-SO_3H$ radical.
- X is an oxygen or sulphur atom or a group -NR-, R being a hydrogen atom or a linear or branched alkyl radical containing 1 to 6 carbon atoms.

**23.** Composition according to Claim 22, characterized in that, in the formula (V), X denotes a -NH- radical, $R_{11}$ denotes a $-SO_3H$ radical, and both $R_{12}$ and $R_{13}$ denote a hydrogen atom.

**24.** Composition according to Claim 1, characterized in that the hydrophilic agent which screens ultraviolet radiation is a compound of the formula (VI)

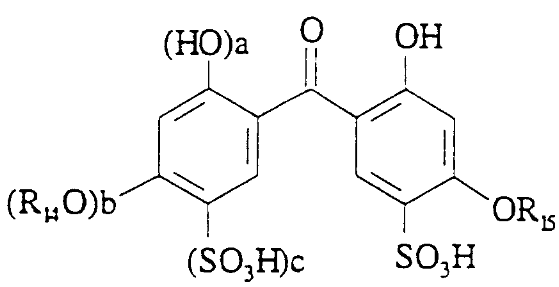

(VI)

in which

- $R_{14}$ and $R_{15}$, which are identical or different, denote a hydrogen atom or a linear or branched alkyl radical containing 1 to 8 carbon atoms
- a, b and c, which are identical or different, are equal to 0 or 1.

**25.** Composition according to Claim 24, characterized in that, in the formula (VI), $a = b = c = 0$ and $R_{15}$ denotes a

methyl radical.

**26.** Composition according to Claim 1, characterized in that the hydrophilic agent which screens ultraviolet radiation is a compound of the formula (VII)

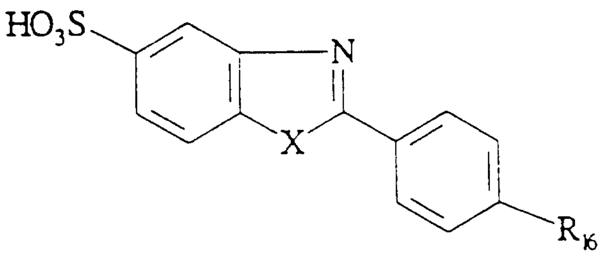

( VII )

in which

- X denotes an oxygen atom or a -NH- radical
- $R_{16}$ denotes a hydrogen atom, a linear or branched, alkyl or alkoxy radical containing 1 to 8 carbon atoms or a group of formula (VIII)

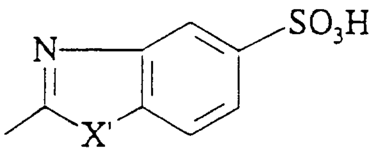

( VIII )

in which X', independently of X, denotes an oxygen atom or a -NH- radical.

**27.** Composition according to Claim 26, characterized in that, in the formula (VII), X denotes the -NH- radical and $R_{16}$ denotes a hydrogen atom.

**28.** Composition according to Claim 26, characterized in that, in the formula (VII), X denotes the -NH-radical, $R_{16}$ denotes the group of formula (VIII) with X' denoting the -NH- radical.

**29.** Composition according to Claim 26, characterized in that, in the formula (VII), X denotes an oxygen atom, $R_{16}$ denotes the group of formula (VIII) with X' denoting an oxygen atom.

**30.** Composition according to any one of Claims 1 to 29, characterized in that the amino-functional silicone derivative has an amine index of greater than 0.25 meq/gram.

**31.** Composition according to Claim 30, characterized in that the amino-functional silicone derivative has an amine index of greater than 0.50 meq/gram.

**32.** Composition according to any one of Claims 1 to 31, characterized in that the amino-functional silicone derivative is a silicone derivative comprising a primary, secondary or tertiary amine function.

**33.** Composition according to Claim 32, characterized in that the silicone derivative comprises a primary amine function $NH_2$ which is linked to the principal chain via a pendant group, or in the $\alpha$ and $\omega$ position on the principal chain.

**34.** Composition according to Claim 32, characterized in that the silicone derivative comprising a secondary amine function is an aminobispropyldimethicone compound.

**35.** Composition according to Claim 32, characterized in that the silicone derivative comprising a tertiary amine function is the compound of structure

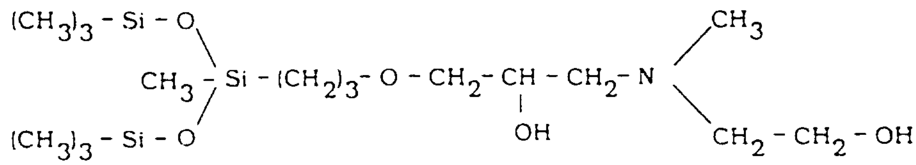

**36.** Composition according to any one of the preceding claims, characterized in that it contains from 0.2 to 10% by weight of the hydrophilic agent which screens ultraviolet radiation, and preferably from 0.5 to 5%.

**37.** Composition according to any one of the preceding claims, characterized in that it contains the amino-functional silicone derivative in a proportion which is necessary for the neutralization of at least 50% of the sulphonic acid functions of the hydrophilic screening agent.

**38.** Composition according to any one of the preceding claims, characterized in that it contains the amino-functional silicone derivative in a proportion which is necessary for the neutralization of 100% of the sulphonic acid functions of the hydrophilic screening agent.

**39.** Composition according to any one of the preceding claims, characterized in that it additionally contains one or more additional hydrophilic or lipophilic UVB and/or UVA sunscreens other than the acidic hydrophilic screening agents according to Claim 1.

**40.** Composition according to Claim 39, characterized in that the additional sunscreens are chosen from cinnamates, salicylates, benzylidenecamphor derivatives, triazine derivatives, benzophenone derivatives, dibenzoylmethane derivatives, β,β-diphenylacrylate derivatives, p-aminobenzoic acid derivatives, menthyl anthranilate, polymer screening agents and silicone screening agents.

**41.** Composition according to Claim 40, characterized in that the additional sunscreens are chosen from the group consisting of 4-tert-butyl-4'-methoxydibenzoylmethane, octyl 2-cyano-3,3-diphenyl-2-propenoate and 3-(4-methylbenzylidene)camphor.

**42.** Composition according to Claim 40, characterized in that the additional sunscreens are chosen from the group consisting of 2-ethylhexyl p-methoxycinnamate, 2,4,6-tris[p-(2'ethylhexyl-1'-oxycarbonyl)anilino]-1,3,5-triazine and octyl 2-cyano-3,3-diphenyl-2-propenoate.

**43.** Composition according to any one of the preceding claims, characterized in that it additionally contains coated or non-coated metal oxide nanopigments.

**44.** Composition according to any one of the preceding claims, characterized in that it additionally comprises cosmetic adjuvants chosen from fats, organic solvents, nonionic thickeners, emollients, antioxidants, opacifying agents, stabilizers, silicones other than the amino-functional silicone derivatives, anti-foaming agents, moisturizers, fragrances, preservatives, nonionic surfactants, fillers, sequestering agents, nonionic polymers, propellants, basifying or acidifying agents and dyes.

**45.** Composition according to any one of the preceding claims, characterized in that it constitutes a protective composition for the human skin or an anti-sun composition and is presented in the form of a nonionic vesicle dispersion, emulsions, cream, milk, gel, cream gel, suspensions, dispersions, powder, solid stick, mousse or spray.

**46.** Composition according to any one of Claims 1 to 44, characterized in that it constitutes a make-up composition for the eyelashes, eyebrows or skin and is presented in a solid or paste form, in an anhydrous or aqueous form, as an emulsion, suspension or dispersion.

**47.** Composition according to any one of Claims 1 to 44 which is used for the protection of hair against ultraviolet radiation, characterized in that it is presented in the form of a shampoo, lotion, gel, emulsion, nonionic vesicle dispersion or hair lacquer.

**48.** Composition according to any one of the preceding claims, characterized in that the said cosmetically acceptable vehicle is an emulsion of the oil-in-water type.

**49.** Method of cosmetic treatment of the skin and/or hair in order to protect them against the effects of UV radiation with wavelengths of between 280 and 400 nm, characterized in that it consists in applying an effective quantity of a substantive screening cosmetic composition as defined in any one of Claims 1 to 48.

**50.** Screening combination comprising, on the one hand, a hydrophilic screening agent containing at least one sulphonic acid radical according to any one of Claims 1 to 29 and, on the other hand, an amino-functional silicone derivative according to any one of Claims 30 to 35.

**51.** Combination according to Claim 50, characterized in that the amino-functional silicone derivative is present in a proportion which is necessary for the neutralization of at least 50% of the sulphonic acid functions of the hydrophilic screening agent.

**52.** Combination according to Claim 50 or 51, characterized in that the amino-functional silicone derivative is present in a proportion which is necessary for the neutralization of 100% of the sulphonic acid functions of the hydrophilic screening agent.

**53.** Use of the combination according to any one of Claims 50 to 52 for the preparation of cosmetic compositions.

## Patentansprüche

**1.** Kosmetische Filterzusammensetzungen,
dadurch gekennzeichnet, daß
sie in einem kosmetisch akzeptablen Träger mindestens ein hydrophiles Mittel, das UV-Strahlung filtert und mindestens eine Sulfonsäuregruppe $-SO_3H$ aufweist, und mindestens ein aminofunktionelles Siliconderivat enthalten.

**2.** Zusammensetzungen nach Anspruch 1, dadurch gekennzeichnet, daß das hydrophile Mittel, das die UV-Strahlung filtert, eine Verbindung mit 3-Benzyliden-2-bornanon-struktur ist.

**3.** Zusammensetzungen nach Anspruch 2, dadurch gekennzeichnet, daß das hydrophile Mittel, das die UV-Strahlung filtert, der folgenden Formel (I) entspricht

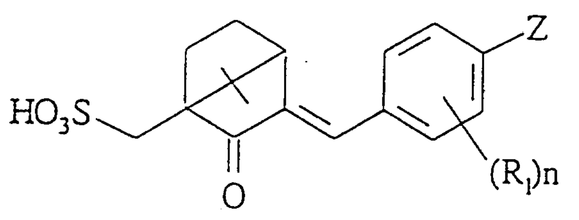

(I)

worin bedeuten:

- Z die Gruppe der Formel

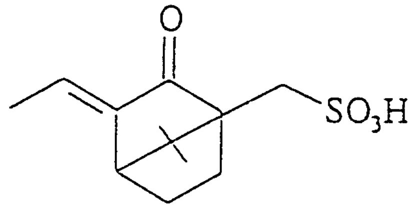

- n 0 oder eine ganze Zahl größer oder gleich 1 und kleiner oder gleich 4,
- $R_1$ oder mehrere geradkettige oder verzweigte Alkyl- oder Alkoxygruppen mit 1 bis 4 Kohlenstoffatomen, die identisch oder voneinander verschieden sind.

**4.** Zusammensetzungen nach Anspruch 3, dadurch gekennzeichnet, daß die Verbindung der Formel (I) Benzol-1,4-[di(3-methylidencampher-10-sulfonsäure)] ist.

**5.** Zusammensetzung nach Anspruch 2, dadurch gekennzeichnet, daß das hydrophile Mittel, das die UV-Strahlung filtert, der folgenden Formel (II) entspricht:

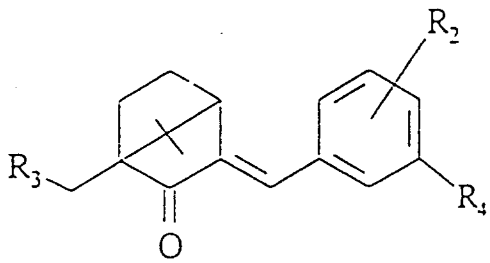

(II)

worin bedeuten:

- $R_2$ ein Wasserstoffatom, ein Halogenatom, eine Alkylgruppe mit 1 bis 4 Kohlenstoffatomen oder die Gruppe -$SO_3H$,

- $R_3$ und $R_4$ ein Wasserstoffatom oder die Gruppe -$SO_3H$, wobei mindestens eine der Gruppen $R_2$, $R_3$ oder $R_4$ die Gruppe -$SO_3H$ bedeutet und $R_2$ und $R_4$ nicht gleichzeitig die Gruppe $SO_3H$ bedeuten können.

**6.** Zusammensetzungen nach Anspruch 5, dadurch gekennzeichnet, daß in der Formel (II) $R_2$ die Gruppe -$SO_3H$ in p-Stellung des Benzylidencamphers und $R_3$ und $R_4$ jeweils ein Wasserstoffatom bedeuten.

**7.** Zusammensetzungen nach Anspruch 5, dadurch gekennzeichnet, daß in der Formel (II) $R_2$ und $R_4$ jeweils Wasserstoff und $R_3$ -$SO_3H$ bedeutet.

**8.** Zusammensetzung nach Anspruch 5, dadurch gekennzeichnet, daß in der Formel (II) $R_2$ eine Methylgruppe in p-Stellung des Benzylidencamphers, $R_4$ die Gruppe -$SO_3H$ und $R_3$ ein Wasserstoffatom bedeutet.

**9.** Zusammensetzung nach Anspruch 5, dadurch gekennzeichnet, daß in der Formel (II) $R_2$ ein Chloratom in p-Stellung des Benzylidencamphers, $R_4$ die Gruppe -$SO_3H$ und $R_3$ ein Wasserstoffatom bedeutet.

**10.** Zusammensetzungen nach Anspruch 5, dadurch gekennzeichnet, daß in der Formel (II) $R_2$ Methyl in p-Stellung des Benzylidencamphers, $R_4$ Wasserstoff und $R_3$ die Gruppe -$SO_3H$ bedeutet.

**11.** Zusammensetzungen nach Anspruch 2, dadurch gekennzeichnet, daß das hydrophile Mittel, das die UV-Strahlung filtert, der folgenden Formel (III) entspricht:

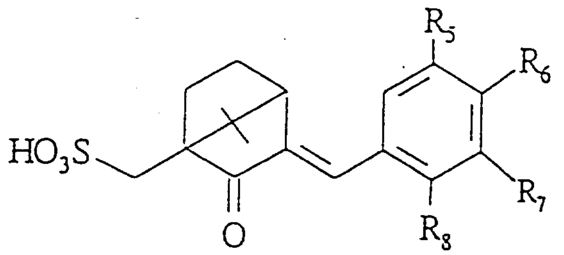

(III)

worin bedeuten:

- $R_5$ und $R_7$, die identisch oder voneinander verschieden sind, Wasserstoff, Hydroxy, eine geradkettige oder verzweigte Alkylgruppe mit 1 bis 8 Kohlenstoffatomen oder eine geradkettige oder verzweigte Alkoxygruppe mit 1 bis 8 Kohlenstoffatomen, wobei mindestens eine der Gruppen $R_5$ und $R_7$ Hydroxy, Alkyl oder Alkoxy bedeutet,
- $R_6$ und $R_8$, die identisch oder voneinander verschieden sind, Wasserstoff oder Hydroxy, wobei mindestens eine der Gruppen $R_6$ und $R_8$ Hydroxy bedeutet,
- mit der Maßgabe, daß $R_7$ keine Alkoxygruppe oder kein Wasserstoffatom bedeutet, wenn $R_5$ und $R_8$ Wasserstoff bedeuten und $R_6$ Hydroxy ist.

**12.** Zusammensetzungen nach Anspruch 11, dadurch gekennzeichnet, daß in der Formel (III) $R_5$ Methyl, $R_6$ Wasserstoff, $R_7$ *tert.*-Butyl und $R_8$ Hydroxy bedeutet.

**13.** Zusammensetzungen nach Anspruch 11, dadurch gekennzeichnet, daß in der Formel (III) $R_5$ Methoxy, $R_6$ Wasserstoff, $R_7$ *tert.*-Butyl und $R_8$ Hydroxy bedeutet.

**14.** Zusammensetzungen nach Anspruch 11, dadurch gekennzeichnet, daß in der Formel (III) $R_5$ und $R_7$ jeweils *tert.*-Butyl, $R_6$ Hydroxy und $R_8$ Wasserstoff bedeutet.

**15.** Zusammensetzungen nach Anspruch 2, dadurch gekennzeichnet, daß das hydrophile Mittel, das die UV-Strahlung filtert, der folgenden Formel (IV) entspricht

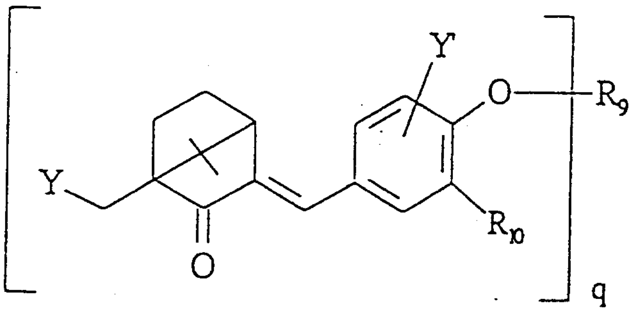

(IV)

worin bedeuten:

- $R_9$ Wasserstoff, eine geradkettige oder verzweigte Alkylgruppe mit 1 bis 18 Kohlenstoffatomen, eine geradkettige oder verzweigte Alkenylgruppe mit 3 bis 18 Kohlenstoffatomen, eine Gruppe, die unter den folgenden Gruppen ausgewählt ist:

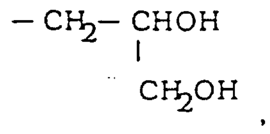

$-(CH_2CH_2O)_n-H$, oder $-CH_2-CHOH-CH_3$ oder eine zweiwertige Gruppe $-(CH_2)_m$ oder $-CH_2-CHOH-CH_2-$, wobei n eine ganze Zahl im Bereich von 1 bis 6 ($1 \leq n \leq 6$) und m eine ganze Zahl im Bereich von 1 bis 10 ($1 \leq n \leq 10$) bedeutet,

- $R_{10}$ Wasserstoff, eine Alkoxygruppe mit 1 bis 4 Kohlenstoffatomen oder die zweiwertige Gruppe -O-, die an die Gruppe $R_9$ gebunden ist, wenn diese ebenfalls zweiwertig vorliegt,
- q eine ganze Zahl 1 oder 2, mit der Maßgabe, daß $R_9$ eine zweiwertige Gruppe bedeuten muß, wenn q 2 ist,
- Y und Y' Wasserstoff oder die Gruppe $-SO_3H$, wobei mindestens eine der Gruppen Y oder Y' von Wasserstoff verschieden ist.

**16.** Zusammensetzungen nach Anspruch 15, dadurch gekennzeichnet, daß in der Formel (IV) q 1 ist, Y, $R_{10}$ jeweils Wasserstoff, $R_9$ Methyl und Y' in 3-Stellung die Gruppe $-SO_3H$ bedeuten.

**17.** Zusammensetzungen nach Anspruch 15, dadurch gekennzeichnet, daß in der Formel (IV) q 1 ist, Y die Gruppe $-SO_3H$, Y' Wasserstoff und $R_{10}$ eine zweiwertige Gruppe -O- bedeutet, die an $R_9$ gebunden ist, welche Methylen bedeutet.

**18.** Zusammensetzungen nach Anspruch 15, dadurch gekennzeichnet, daß in der Formel (IV) q 1 ist, Y $-SO_3H$, Y' und $R_{10}$ jeweils ein Wasserstoffatom und $R_9$ Methyl bedeutet.

**19.** Zusammensetzungen nach Anspruch 15, dadurch gekennzeichnet, daß in der Formel (IV) q 1 ist, Y die Gruppe $-SO_3H$, Y' Wasserstoff, $R_9$ Methyl und $R_{10}$ Methoxy bedeutet.

**20.** Zusammensetzungen nach Anspruch 15, dadurch gekennzeichnet, daß in der Formel (IV) q 1 ist, Y die Gruppe $-SO_3H$, Y' und $R_{10}$ jeweils ein Wasserstoffatom und $R_9$ n-Butyl bedeutet.

**21.** Zusammensetzungen nach Anspruch 15, dadurch gekennzeichnet, daß in der Formel (IV) q 1 ist, Y die Gruppe $-SO_3H$, Y' ein Wasserstoffatom, $R_9$ n-Butyl und $R_{10}$ Methoxy bedeutet.

**22.** Zusammensetzungen nach Anspruch 2, dadurch gekennzeichnet, daß das hydrophile Mittel, das die UV-Strahlung filtert, der folgenden Formel (V) entspricht:

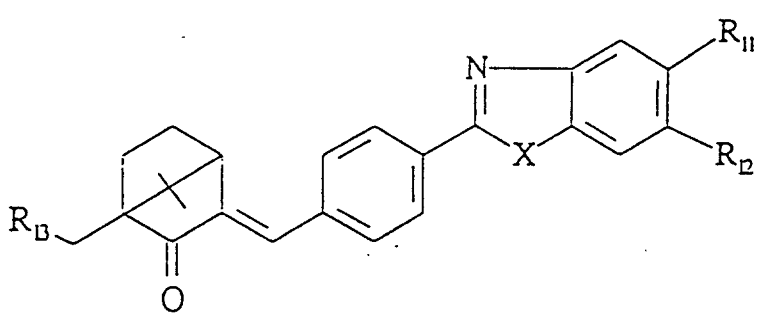

(V)

worin bedeuten:

- $R_{11}$ Wasserstoff, eine geradkettige oder verzweigte Alkyl- oder Alkoxygruppe mit 1 bis 6 Kohlenstoffatomen

oder die Gruppe $-SO_3H$,

- $R_{12}$ Wasserstoff, eine geradkettige oder verzweigte Alkyl- oder Alkoxygruppe mit 1 bis 6 Kohlenstoffatomen,
- $R_{13}$ Wasserstoff oder die Gruppe $-SO_3H$, wobei mindestens eine der Gruppen $R_{11}$ oder $R_{12}$ $-SO_3H$ bedeutet,
- X Sauerstoff oder Schwefel oder eine Gruppe -NR-,
- wobei R Wasserstoff oder eine geradkettige oder verzweigte Alkylgruppe mit 1 bis 6 Kohlenstoffatomen ist.

**23.** Zusammensetzungen nach Anspruch 22, dadurch gekennzeichnet, daß in der Formel (V) X die Gruppe -NH-, $R_{11}$ die Gruppe $-SO_3H$ und $R_{12}$ und $R_{13}$ beide ein Wasserstoffatom bedeuten.

**24.** Zusammensetzungen nach Anspruch 1, dadurch gekennzeichnet, daß das hydrophile Mittel, das die UV-Strahlung filtert, eine Verbindung der Formel (VI) ist

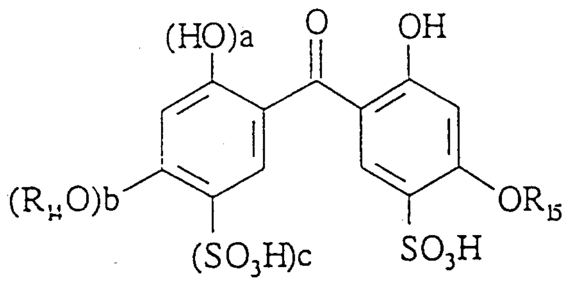

(VI)

worin bedeuten:

- $R_{14}$ und $R_{15}$, die identisch oder voneinander verschieden sind, Wasserstoff oder eine geradkettige oder verzweigte Alkylgruppe mit 1 bis 8 Kohlenstoffatomen
- a, b und c, die identisch oder voneinander verschieden sind, 0 oder 1.

**25.** Zusammensetzungen nach Anspruch 24, dadurch gekennzeichnet, daß in der Formel (VI) a=b=c=0 und $R_{15}$ eine Methylgruppe bedeutet.

**26.** Zusammensetzungen nach Anspruch 1, dadurch gekennzeichnet, daß das hydrophile Mittel, das die UV-Strahlung filtert, eine Verbindung der Formel (VII) ist

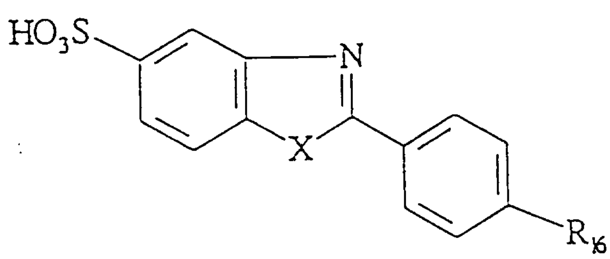

(VII),

worin bedeuten:

- X Sauerstoff oder die Gruppe -NH-;
- $R_{16}$ ein Wasserstoffatom, eine geradkettige oder verzweigte Alkyl- oder Alkoxygruppe mit 1 bis 8 Kohlenstoffatomen oder eine Gruppe der Formel

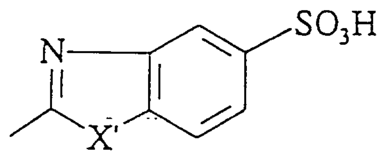

(VIII),

worin X' Sauerstoff oder die Gruppe -NH- bedeutet.

**27.** Zusammensetzungen nach Anspruch 26, dadurch gekennzeichnet, daß in der Formel (VII) X die Gruppe -NH- und $R_{16}$ Wasserstoff bedeutet.

**28.** Zusammensetzungen nach Anspruch 26, dadurch gekennzeichnet, daß in der Formel (VII) X die Gruppe -NH- und $R_{16}$ die Gruppe der Formel (VIII) bedeutet, wobei in der Formel (VIII) X' die Gruppe -NH- ist.

**29.** Zusammensetzungen nach Anspruch 26, dadurch gekennzeichnet, daß in der Formel (VII) X Sauerstoff und $R_{16}$ die Gruppe der Formel (VIII) bedeutet, wobei X' in der Formel (VIII) Sauerstoff ist.

**30.** Zusammensetzungen nach einem der Ansprüche 1 bis 29, dadurch gekennzeichnet, daß das aminofunktionelle Siliconderivat einen Aminoindex über 0,25 meq/g aufweist.

**31.** Zusammensetzungen nach Anspruch 30, dadurch gekennzeichnet, daß das aminofunktionelle Siliconderivat einen Aminoindex über 0,5 meq/g aufweist.

**32.** Zusammensetzungen nach einem der Ansprüche 1 bis 31, dadurch gekennzeichnet, daß das aminofunktionelle Siliconderivat ein Siliconderivat ist, das eine primäre, sekundäre oder tertiäre Aminogruppe aufweist.

**33.** Zusammensetzungen nach Anspruch 32, dadurch gekennzeichnet, daß das Siliconderivat eine primäre Aminogruppe $NH_2$ aufweist, die über eine Gruppe an die Hauptkette gebunden ist oder sich in $\alpha$- und $\omega$-Stellung an der Hauptkette befindet.

**34.** Zusammensetzungen nach Anspruch 32, dadurch gekennzeichnet, daß das Siliconderivat mit sekundärer Aminogruppe eine Aminobispropyldimethicon-Verbindung ist.

**35.** Zusammensetzungen nach Anspruch 32, dadurch gekennzeichnet, daß das Siliconderivat mit tertiärer Aminogruppe die Verbindung der folgenden Struktur ist:

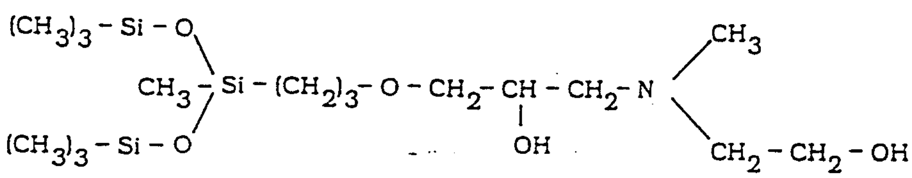

**36.** Zusammensetzungen nach einem der vorhergehenden Ansprüche, dadurch gekennzeichnet, daß sie 0,2 bis 10 Gew.-% hydrophiles Mittel, das die UV-Strahlung filtert, und vorzugsweise 0,5 bis 5 % enthält.

**37.** Zusammensetzungen nach einem der vorhergehenden Ansprüche, dadurch gekennzeichnet, daß sie das aminofunktionelle Siliconderivat in einem Mengenanteil enthalten, der erforderlich ist, um mindestens 50 % der Sulfonsäuregruppen des hydrophilen Filters zu neutralisieren.

**38.** Zusammensetzungen nach einem der vorhergehenden Ansprüche, dadurch gekennzeichnet, daß sie das aminofunktionelle Siliconderivat in einem Mengenanteil enthalten, der erforderlich ist, um 100 % der Sulfonsäuregruppen

des hydrophilen Filters zu neutralisieren.

**39.** Zusammensetzungen nach einem der vorhergehenden Ansprüche, dadurch gekennzeichnet, daß sie ferner einen oder mehrere zusätzliche im UV-B- oder UV-A-Bereich wirksame, hydrophile oder lipophile Filter enthalten, die von den sauren hydrophilen Filtern nach Anspruch 1 verschieden sind.

**40.** Zusammensetzungen nach Anspruch 39, dadurch gekennzeichnet, daß die zusätzlichen Sonnenschutzfilter unter Cinnamaten, Salicylaten, Benzylidencampherderivaten, Triazinderivaten, Benzophenonderivaten, Dibenzoylmethanderivaten, β,β-Diphenylacrylatderivaten, p-Aminobenzoesäurederivaten, Menthylanthranilat, Filterpolymeren und Siliconfiltern ausgewählt sind.

**41.** Zusammensetzungen nach Anspruch 40, dadurch gekennzeichnet, daß die zusätzlichen Sonnenschutzfilter unter 4-*tert.*-Butyl-4'-methoxydibenzoylmethan, Octyl-2-cyano-3,3-diphenyl-2-propeonat und 3-(4-Methylbenzyliden)-campher ausgewählt sind.

**42.** Zusammensetzungen nach Anspruch 40, dadurch gekennzeichnet, daß die zusätzlichen Sonnenschutzfilter unter 2-Ethylhexyl-p-methoxycinnamat, 2,4,6-Tris[p-(2'-Ethylhexyl-1'-oxycarbonyl)-anilino-1,3,5-triazin und Octyl-2-cyano-3,3-diphenyl-2-propenoat ausgewählt sind.

**43.** Zusammensetzungen nach einem der vorhergehenden Ansprüche, dadurch gekennzeichnet, daß sie ferner Nanopigmente von Metalloxiden, die gegebenenfalls umhüllt sind, enthalten.

**44.** Zusammensetzungen nach einem der vorhergehenden Ansprüche, dadurch gekennzeichnet, daß sie ferner kosmetische Hilfsstoffe enthalten, die unter Fettsubstanzen, organischen Lösungsmitteln, nichtionischen Verdickungsmitteln, reizlindernden Mitteln, Antioxidantien, Trübungsmitteln, Stabilisierungsmitteln, Siliconen, die von den aminofunktionellen Siliconderivaten verschieden sind, Antischaummitteln, Hydratisierungsmitteln, Parfums, Konservierungsmitteln, nichtionischen grenzflächenaktiven Stoffen, Füllstoffen, Maskierungsmitteln, nichtionischen Polymeren, Treibmitteln, Mitteln zum Alkalischmachen oder Ansäuern und Färbemitteln ausgewählt sind.

**45.** Zusammensetzungen nach einem der vorhergehenden Ansprüche, dadurch gekennzeichnet, daß sie Zusammensetzungen zum Schutz der menschlichen Epidermis und Sonnenschutzmittel darstellen und in Form von nichtionischen Vesikeldispersionen, Emulsionen, Cremes, Milchen, Gelen, Gelcremes, Suspensionen, Dispersionen, Pulvern, festen Stiften, Schäumen oder Sprays vorliegen.

**46.** Zusammensetzungen nach einem der Ansprüche 1 bis 44, dadurch gekennzeichnet, daß sie Zusammensetzungen zum Schminken der Wimpern, Augenbrauen oder der Haut darstellen und in fester oder pastöser, wasserfreier oder wäßriger Form, als Emulsion, Suspension oder Dispersion vorliegen.

**47.** Zusammensetzungen nach einem der Ansprüche 1 bis 44, die zum Schutz der Haare gegen UV-Strahlung verwendet werden, dadurch gekennzeichnet, daß sie als Haarwaschmittel, Lotion, Gel, Emulsion, nichtionische Vesikeldispersion oder Haarlack vorliegen.

**48.** Zusammensetzung nach einem der vorhergehenden Ansprüche, dadurch gekennzeichnet, daß der kosmetisch akzeptable Träger eine Öl-in-Wasser-Emulsion ist.

**49.** Verfahren zur kosmetischen Behandlung der Haut oder der Haare, um sie gegen die Wirkungen der UV-Strahlung mit einer Wellenlänge im Bereich von 280 bis 400 nm zu schützen, dadurch gekennzeichnet, daß es darin besteht, eine wirksame Menge einer substantiven kosmetischen Filterzusammensetzung nach einem der Ansprüche 1 bis 48 aufzutragen.

**50.** Filterkombination, die ein hydrophiles Filter mit mindestens einer Sulfonsäuregruppe nach einem der Ansprüche 1 bis 29 und ein aminofunktionelles Siliconderivat nach einem der Ansprüche 30 bis 35 enthält.

**51.** Kombination nach Anspruch 50, dadurch gekennzeichnet, daß das aminofunktionelle Siliconderivat in einem Mengenanteil vorliegt, der erforderlich ist, um mindestens 50 % der Sulfonsäuregruppen des hydrophilen Filters zu neutralisieren.

**52.** Kombination nach Anspruch 50 oder 51, dadurch gekennzeichnet, daß das aminofunktionelle Siliconderivat in

einem Mengenanteil vorliegt, der erforderlich ist, um 100 % der Sulfonsäuregruppen des hydrophilen Filters zu neutralisieren.

**53.** Verwendung der Kombination nach einem der Ansprüche 50 bis 52 zur Herstellung von kosmetischen Zusammensetzungen.